# EUROPEAN PATENT SPECIFICATION

(11) **EP 1 523 291 B1**
(45) Date of publication and mention of the grant of the patent: **18.06.2008**
(21) Application number: 03764417.6
(22) Date of filing: 10.07.2003
(51) Int. Cl.: A61F 2/46, A61B 17/16

(54) **KIT FOR INTERPOSITIONAL ARTHROPLASTY**
VORRICHTUNG ZUR ZWISCHENPOSITIONIERUNGS-ARTHROPLASTIK
TROUSSE POUR ARTHROPLASTIE AVEC INTERPOSITION

(30) Priority: 11.07.2002 US 395301 P; 19.12.2002 US 40883; 22.01.2003 US 2142
(43) Date of publication of application: 20.04.2005
(73) Proprietor: ADVANCED BIO SURFACES, INC., Minnetonka, MN 55345 (US)
(72) Inventor: FELT, Jeffrey, C., Greenwood, MN 55331 (US); RYDELL, Mark, A., Golden Valley, MN 55416 (US); GRIFFIN, David, Vero Beach, FL 32960 (US); BUSCEMI, Paul, J., Long Lake, MN 55356 (US); ARSENYEV, Alexander, Eagan, MN 55122 (US)
(74) Representative: Walker, Ross Thomson
(86) International application number: PCT/US2003/021513
(87) International publication number: WO 2004/006811

(56) References cited:
- EP-A- 0 378 002
- WO-A-00/59411
- WO-A-01/66021
- US-A- 5 725 531
- US-A1- 2001 037 114
- US-B1- 6 224 630

## Description

### TECHNICAL FIELD

The invention relates to kits that include instruments for use in preparing (e.g., smoothing) and/or using (e.g., selecting and implanting) interpositional implants as described herein. The closest prior art is WO 0059411A, which discloses an interpositional implant system.

### BACKGROUND OF THE INVENTION

Applicant has previously described, *inter alia,* prosthetic implants formed of biomaterials that can be delivered and finally cured *in situ*, and/or that can be partially or fully prepared *ex vivo*, for implantation into the body, e.g., using minimally invasive techniques. See for instance, U.S. Patent Nos. 5,556,429; 5,795,353; 5,888,220; 6,079,868; 6,140,452; 6,224,630; 6,248,131; 6,306,177; and 6,443,988, as well as US Application Publication Nos. US-2002-0156531; US-2002-0127264; US-2002-0183850; and US-2002-0173852, and International applications having Publication Nos. WO 95/30388; WO 97/26847; WO 98/20939; WO 99/44509; WO 02/17821; and WO 02/17825.

US Patent No. 6,206,927 describes a self-centering meniscal prosthesis device suitable for minimally invasive, surgical implantation into the cavity between a femoral condyle and the corresponding tibial plateau is composed of a hard, high modulus material shaped such that the contour of the device and the natural articulation of the knee exerts a restoring force on the free-floating device. In what appears to be a related manner, Sulzer has introduced a unicompartmental interpositional spacer to treat osteoarthritis in the knee. See "Little Device Could Pack a Big Punch", Sulzer Medica Journal Edition 2/2000 (www.sulzermedica.com/media/smj-full-tex/2000/0002-full-text-6.html). The device is described as a metallic kidney-shaped insert which fills in for the damaged cartilage between the femur and the tibia. See also more recently issued US Patent No. 6,558,421 (Fell et al) and corresponding published applications having Serial Nos. 2003/0060882 (Fell et al); 2003/0060883 (Fell et al); 2003/0060884 (Fell et al); 2003/0060885 (Fell et al); and 2003/0060888 (Fell et al).

On another topic, over recent years, a variety of devices and systems have been developed and introduced for use in minimally invasive surgery, including orthopedic surgery. An array of orthopedic instruments are manufactured by companies such as MicroAire, Stryker, Zimmer/Hall, Aesculap, Codman, 3M, and Dyonics.

Generally, such cutting and shaping devices are used in open surgical procedures, e.g., for the purpose of resecting bone in order to provide partial or total knee replacements. See, for instance, Spotorno, et al., US Patent No. 6,319,256, which describes a bone rasp for a femur head prosthesis. See also, Braslow, et al., US Patent No. 6,059,831, which describes a method of implanting a uni-condylar knee prosthesis, including the steps of preparing the bone surfaces of both the femoral and tibal compartments. The femoral compartment is prepared by making a distal cut, a posterior cut, and a posterior chamfer cut. The tibial compartment is prepared by using a cutting guide and following the sclerotic bone formation on the proximal tibia. See also, Engh, et al., which describes an apparatus and method for "sculpting" the surface of a joint.

Surgical orthopedic instruments can also include arthroscopic and other minimally invasive instruments such as reciprocating bone saws, rasps, and the like. For instance, Shechter et al. (US Patent No. 5,685,840) describes a method and apparatus for minimally invasive tissue removal that includes motor driven reciprocating cutting blade, having the ability to control the frequency of reciprocation using an integrated feedback control system, and including optional rasp or tissue morcelator cutting heads.

Surgical, including minimally invasive, devices have also been described to achieve bone cutting or smoothing using non-mechanical means, as by the use of lasers for instance. See, for instance, "Parameters for Safe Application of the 2.1 µm Holmium:YAG Laser for Chondroplasty of the Medial Femoral Condyle", Janecki et al., Arthroplasty Arthroscopic Surgery 9(1):1-6, 1998.

On yet another topic, a variety of devices exist for use in performing various spatial measurements in the course of surgery, and particularly orthopedic surgery. With particular attention on the knee, most measuring devices are designed for either external use, as in segmental measurements of the knee, or for use in the course of open surgery, and particularly for total knee replacement.

Externally, segmental measurements can be made of various orthopedic dimensions. See, for instance, "Segmental Measures" at http://www.people.virginia.edu/∼smb4v/growth/segmenta.htm, which describes the manner in which knee height can be used to estimate stature in someone with contractures who is unable to straighten out. The subject can be either lying supine on a table or sitting upright. The subject's knee and ankle should both be at a ninety degree angles. A caliper is used for this measurement. One end of the caliper is placed under the heel of the foot right under the malleolus, and the other blade of the caliper is placed on the anterior surface of the thigh approximately above the head of the fibula. This will usually be one or one and one half inches behind the proximal edge of the patella. Slight pressure should be applied for an accurate measurement, and the shaft of the caliper should be aligned with the long axis of the leg. The measurement is then read and recorded to the nearest 0.1 cm.

Similarly, tibial length can be measured from the medial joint line of the knee to the distal edge of the medial malleolus. The subject should be sitting with the leg to be measured crossed over the other leg. The measurer should locate and mark the two important landmarks on the subject. First, the medial epicondyle of the femur should be found and a mark made on the subject's skin at the medial facet of the femorotibial joint space. Second, the distal tip of the malleolus should be found and marked. The measurer should sit or squat next to the leg to obtain an accurate measurement. The arms or blades of the anthropometer are placed on both landmarks, and a measurement is read. The shaft of the anthropometer should be parallel to the axis of the leg. This measurement can also be taken with a flexible measuring tape in which the zero end is placed on the malleolus landmark and the measurement value is read on the proximal tibial border. The measurement is taken to the nearest 0.1 cm.

A representative example of the measurements made in the course of total knee replacement can be found at US Patent No. 4,736,737, which describes a tibial cutting jig for use in obtaining accurate tibial resection in the course of a total knee prosthesis implantation procedure. The tibial cutting jig includes a base for sliding reception onto an intramedullary alignment rod preinstalled generally along the longitudinal axis of the tibia. The base includes laterally extending outriggers carrying removable measurement keys of selected size for spacing the base above the tibial plateau by a selected dimension. An anterior saw guide depends from the base and is thus positioned relative to the tibial plateau in accordance with the sizes of the measurement keys.

On yet another topic, See, for instance, M. Wiklund, "Eleven Keys to Designing Error-Resistant Medical Device", in MDDI (May 2002), also at http://www.devicelink.com/mddi/archive/02/05/004.html, which highlights the importance of providing medical devices which reduce the likelihood that errors will occur.

In spite of developments to date, there remains a need for a joint prosthesis system for interpositional arthroplasty that provides an optimal combination of properties such as ease of preparation and use, and performance within the body. There particularly remains a need for instruments and components, and corresponding kits containing and integrating such instruments and components, for use by surgeons in the course of selecting and implanting such interpositional implants.

### SUMMARY OF THE INVENTION

The present invention relates to devices for treating joints that have deteriorated to the "bone on bone" stage. Devices are provided for providing some or all of the steps of: a) preparing a joint to receive an implant, b) determining an appropriate implant size for a particular joint, c) determining an appropriate implant thickness, d) inserting the implant into the joint, and/or e) securing the implant within the joint to a desired extent.

An apparatus in accordance with the present invention is provided for determining an optimal size for an implant to be inserted into the joint. In a particularly preferred embodiment, as described below, the implant is designed to provide a glide path with respect to the femoral condyle. Such a device can be used in patients having joints that have progressed to the stage of "bone on bone", and thus provides a replacement for the function of articular cartilage as well as some or all of the meniscus, and particularly at the central weight-bearing area of the medial or lateral tibial plateau, in order to restore alignment, while providing an elastomeric, cushioning function. In turn, the present implant is more permanently anchored in place, in significant part by one or more posterior projections, such as the posterior lip, as well by the optional but preferred use of anterior fixation means (such as embedded sutures) secured to anterior soft tissue structures.

In one embodiment, a preferred implant in accordance with the present invention provides a unique combination of a femoral glide path and convexity of the tibial surface of the implant, together with a posterior mesial lip. In turn, the implant provides an indentation adapted to accommodate the tibial spine, which together with a slight feathering of the implant on the underside at the tibial spine, the general kidney shape of the implant, and the convexity of the tibial surface, will permit the implant to be congruent with the concave tibia and the posterior mesial lip that extends over the posterior portion of the tibia and into the mesial side of the tibia into the PCL fossa of the tibia. Importantly, such an implant can be provided in various sizes to accommodate different anterior-posterior dimensions of the tibia and different tibial concavities. In other words, the amount of convexity of the tibial surface will be varied with the different sizes depending on the amount of actual concavity that there is in the tibia. In some applications, however, applicant has found that "one size fits all" with respect to tibial concavity. Selection of an optimal size (and optionally also geometry) is facilitated by use of a measuring device of the present invention.

A kit of the present invention preferably includes a device for measuring one or more dimensions associated with the knee, and has particular use for measuring various aspects associated with the tibial plateau of the medial compartment of the knee in the course of preparing and/or sizing interpositional implants. The device is particularly well suited to be used with small incisions (e.g., less than about 7,6 cm (3 inches), and preferably less than about 3,8 cm (1½ inch)) of the type used to perform arthrotomy procedures involving the knee. In another aspect, the invention provides devices for measuring one or more dimensions selected from the group consisting of an anterior-posterior dimension, a medial-lateral dimension, and a height/depth dimension. In a preferred embodiment, the device can be used to determine a dimension between the anterior and posterior edges of the tibial surface, while also providing a suitable depth measurement of the tibial depression (also referred to herein as "bowl") at a point approximately midway between the raised anterior and posterior edges of the tibial plateau. It is preferred to measure at least the anterior-posterior length, since the medial lateral dimension of preferred implants will typically correspond in a predictable fashion with the anterior-posterior dimension.

Generally, that depth is determined as the distance(s) between the bottommost point of the tibial plateau, and a line drawn between the uppermost anterior and posterior portions of the tibial plateau. The device can be calibrated and used in any suitable fashion, e.g., having independent gradations along various axes, or having stable or moveable markings that are unique to and correlate with particular implant selections.

In a particularly preferred embodiment, the present invention includes a device for:
a) measuring the anterior/posterior length of the tibial plateau, and preferably that of the medial compartment of the knee, and also for
b) measuring the depth of the bowl shaped surface of the tibial plateau.

Preferably, the measuring device is of sufficient size and proportions to permit it to be inserted (e.g., turned onto its side) into the same small arthrotomy incision through which the implant itself is to be placed. For instance, in the embodiment shown herein the device is sufficiently thin (ruler like) to permit it to be slipped into the arthrotomy incision and between the distracted condylar and tibial surfaces.

Once appropriate size/ shape have been determined, an appropriate final implant can be selected, implanted and secured. In an additional aspect of the present invention, some or all of the components of this invention can be designed in a manner that eases their selection and use, while serving to minimize error. For example, some or all of the components can be number coded, bar coded, shape coded, tactile coded and/or visually (e.g. color) coded).

An implant in accordance with the present invention can be used in a method that includes first determining the proper implant thickness needed to match physiological values. The surgeon prepares the site arthroscopically, removing excess cartilage and removing the medial meniscus to the medial ring, using a portal of about 1cm in order to provide suitable arthroscopic access while maintaining the presence of fluid in the joint. The implant can be initially molded ex vivo and include one or more embedded or attached fixation portions (e.g., anterior sutures or tabs), at which time it is inserted into the knee. The surgeon will then typically feel the implant once in position, to confirm that the implant is properly seated, and will extend the knee to provide varus stress on the lower leg, obtaining congruency as the implant continues to cure by finally molding both surfaces of the implant (to both the tibial surface and condyle, respectively).

In the preferred example, the patient will have a diagnosis of osteoarthritis and have loss of cartilage on the articulating surface. A determination will be made of the amount of correction needed for the reestablishment of a normal angle of articulation. The ligaments will be balanced so that there is no loss of range of motion with the implant in place. In some applications the horizontal plane of the original articular surface runs through the center of the implant.

Access to the site is preferably obtained in a minimally invasive way. In a particularly preferred example, this is accomplished through arthroscopic means with arthroscopic portals. In an alternative example, the access is accomplished by a mini arthrotomy with a small incision that allows access to the joint without sacrificing nerves, vessels, muscles or ligaments surrounding the joint. In the preferred embodiment fibrillated articulating cartilage that is degenerated is removed down to the subchondral surface.

A medial arthrotomy is created to provide access for the implant. This also provides an opening to use one or more smoothing devices of the present invention on either the femoral and/or tibial surfaces and completion of the anterior. The smoothing device can be, for example, secured to a powered driver (e.g., a Triton brand reciprocating saw) by inserting the shaft of the device and tightening the collett on the driver. The speed of the driver can be controlled in two ways, namely, by either limiting the air pressure delivered to the driver using an air regulator, and/or by a variable speed valve on the driver, which provides more speed (strokes per second) with increased depression of the control lever.

The smoothing device can be manipulated around and within the joint space, usually guided by placing an index finger on the non-cutting side of the blade. In some advantageous embodiments, blade is sufficiently flexible to permit it to be bended by finger pressure alone, without undue fatigue on the part of the surgeon. Ridges and shape points can be removed from the femur, while taking care not to cut through to trabecular bone. The relatively non-aggressive cutting surface of the device, relative to conventional rasps and rotating burrs, makes this easier to accomplish. Osteophytes should also be removed if they might impinge on the implant or limit range of motion.

Smoothness of the femoral and/or tibial surfaces can be judged in any suitable manner, including by finger palpation. When the surfaces are deemed smooth enough, the joint is thoroughly irrigated to remove any debris. Although typically powered, the excursion can be kept within a range sufficient to permit the surgeon's finger to be kept on the opposite (non-smoothing) surface of the blade-like device, in order to gently oscillate with it. This, combined with the desired flexibility of the device permit it to be moved around the joint, assuming different conformations, in order to smooth any particular surface.

In some advantageous implementations, the body of a smoothing tool is adjusted to the anatomy by bending so it can access areas not accessible with a straight rasp or shaver. For example, the bend allows the smoother to remove osteophytes from the posterior portion of the condyle, which would not be accessible with a commonly used rasp or shaver. The smoother can also be guided into contact with different areas of the bone by flexing and extending the joint. Since the operator need only guide the smoother into position and the motion of the smoother which causes the bone removal is provided by the reciprocating action of the saw, it can easily be used through 1 cm portal as well as a small arthrotomy. Since the abrasive surface is non-aggressive to soft tissue the surgeon can use a gloved indexed finger to direct, enhance and evaluate the smoothing of a bony surface.

### DESCRIPTION OF THE DRAWINGS

Figure 1 is a partial front view of a human skeleton including a left leg and a right leg.
Figure 2 is a diagrammatic representation of a stocking set including a plurality of implants.
Figure 3 is a flow chart illustrating a method in accordance with an exemplary embodiment of the present invention.
Figure 4 is a plan view of a kit in accordance with an exemplary embodiment of the present invention.
Figure 5 is a diagrammatic illustration of an evaluation kit comprising a plurality of implant template kits.
Figure 6 includes a number of views showing an implant template in accordance with the present invention.
Figure 7 is a plan view showing a first implant template, a second implant template, and a third implant template.
Figure 8 is a plan view showing a first trial, a second trial, and a third trial.
Figure 9 is a side view including an implant template shown in cross-section.
Figure 10 is an additional side view illustrating a sizing method in accordance with the present invention.
Figure 11 includes a number of views showing a tibial prep tool in accordance with an exemplary embodiment of the present invention.
Figure 12 is a top view showing tibial prep tool disposed proximate a tibial plateau of a tibia.
Figure 13 is a partial front view of a human skeleton including a left leg and a right leg.
Figure 14 is a perspective view of a femoral prep tool in accordance with an exemplary embodiment of the present invention.
Figure 15 includes a number of views showing a femoral prep tool in accordance with an exemplary embodiment of the present invention.
Figure 16 is an elevation view in which a femoral prep tool is shown in lateral cross-section.
Figure 17 is a side view illustrating femoral prep tool shown in the previous figure.
Figure 18 is an additional side view illustrating an exemplary smoothing step of an exemplary method in accordance with the present invention.
Figure 19 is a front view of a measuring device in accordance with the present invention.
Figure 20 is a rear view of measuring device shown in the previous figure.
Figure 21 is a side view illustrating a measuring method in accordance with an exemplary method of the present invention.
Figure 22 is a perspective view of a gripper in accordance with an exemplary embodiment of the present invention.
Figure 23 is a cross-sectional view of gripper shown in the previous figure.
Figure 24 is an exploded view of gripper shown in the previous figure.
Figure 25 is a side view showing a leg and a gripper.
Figure 26 shows various views of an implant in accordance with an additional exemplary embodiment of the present invention.
Figure 27 shows various views of an implant in accordance with an additional exemplary embodiment of the present invention.
Figure 28 is a top view showing an implant including a plurality of tethers.
Figure 29 is a diagrammatic representation of a stocking set including a plurality of implants.
Figure 30 includes an elevation view and a plan view of an implant in accordance with an additional exemplary embodiment of the present invention.
Figure 31 shows side-by-side views of implants for the left an right knees.
Figure 32 shows three views of an implant in accordance with an exemplary embodiment of the present invention.
Figure 33 shows three additional views of the implant shown in the previous figure.
Figure 34 shows various dimensions relating to an implant in accordance with an exemplary embodiment of the present invention.
Figure 35 includes a number of views showing an implant template 878 in accordance with the present invention.

### DETAILED DESCRIPTION OF THE DRAWING

The following detailed description should be read with reference to the drawings, in which like elements in different drawings are numbered identically. The drawings, which are not necessarily to scale, depict selected embodiments and are not intended to limit the scope of the invention. Examples of constructions, materials, dimensions, and manufacturing processes are provided for selected elements. All other elements employ that which is known to those of skill in the field of the invention. Those skilled in the art will recognize that many of the examples provided have suitable alternatives that can be utilized.

In one preferred embodiment, the system involves the preparation and use of one or more components (e.g., polymeric and/or metallic) that can be at least partially formed outside the body, for insertion and placement into the body, and that can optionally then be further formed within the joint site in order to enhance conformance. The optional ability to finally form one or more components *in situ* provides various additional benefits, such as increased control over the overall size and shape of the final prosthesis, improved shape and compliance of the surface apposing natural bone, and finally, improved shape and compliance of the opposite, articulating surface. The method and system permit the on site preparation or previous manufacture of a unicompartmental interpositional arthroplasty device that comprises a polymeric material such as polyurethane. The components of the system are preferably coordinated, e.g., by being similarly designed and/or labeled, in order to facilitate their use and thereby ensure the proper selection and implantation of an implant.

In a related and particularly preferred embodiment, the implant can be prepared (including full formed and/or cured) *ex vivo,* for later implantation. In a particularly preferred embodiment, as described below, the present invention therefore provides an implant that is designed to be formed to and congruent with the tibial surface, having a final femoral surface shape that serves largely as a glide path with respect to the femoral condyle. Such a device can be used in patients having joints that have progressed to the stage of "bone on bone", and thus provides a replacement for the function of articular cartilage, and optionally some of the natural meniscus, and particularly at the central weight-bearing area, in order to restore alignment, providing an elastomeric, cushioning function. A preferred implant of this type is also congruent with the tibial surface, based upon both its initial shape, together with whatever final shaping may occur *in site*. In turn, the present implant is more permanently anchored in place, in significant part by one or more posterior projections, such as the posterior lip, as well by the optional but preferred use of anterior fixation means (such as, for example, embedded sutures).

In addition, various method steps and components of the kit described herein are considered to be novel in their own right, and include those that can be used in the course of delivering any interpositional arthroplasty device, and in any joint of the body, including those described in the '927 patent identified above.

An implant for use in a kit of the present invention can be can be prepared from any suitable material, including polymeric and non-polymeric (e.g., metallic) and combinations thereof. Typically, the materials include polymeric materials, having an optimal combination of such properties as biocompatibility, physical strength and durability, and compatibility with other components (and/or biomaterials) used in the assembly of a final composite. Examples of suitable materials for use in preparing the preformed component(s) can be the same or different from the *in situ* curing biomaterial, and include polyurethanes, polyethylenes, polypropylenes, Dacrons, polyureas, hydrogels, metals, ceramics, epoxies, polysiloxanes, polyacrylates, as well as biopolymers, such as collagen or collagen-based materials or the like and combinations thereof.

Suitable polyurethanes for use as either the preformed component or biomaterial can be prepared by combining: (1) a prepolymer component (e.g., quasi- or true prepolymer) comprising the reaction product of one or more polyols, and one or more diisocyanates, and optionally, one or more hydrophobic additives, and (2) a curative component comprising one or more polyols, one or more chain extenders, one or more catalysts, and optionally, other ingredients such as an antioxidant, and hydrophobic additive.

In the embodiment in which an *in situ* curing polymer is used, the present invention preferably provides a biomaterial in the form of a curable polyurethane composition comprising a plurality of parts capable of being mixed at the time of use in order to provide a flowable composition and initiate cure, the parts including: (1) a prepolymer component comprising the reaction product of one or more polyols, and one or more diisocyanates, optionally, one or more hydrophobic additives, and (2) a curative component comprising one or more polyols, one or more chain extenders, one or more catalysts, and optionally, other ingredients such as an antioxidant, hydrophobic additive and dye. Upon mixing, the composition is sufficiently flowable to permit it to be delivered to the body, and there be fully cured under physiological conditions. Preferably, the component parts are themselves flowable, or can be rendered flowable, in order to facilitate their mixing and use.

The flowable biomaterial used in this invention preferably includes polyurethane prepolymer components that react either *ex vivo* or *in situ* to form solid polyurethane ("PU"). The formed PU, in turn, includes both hard and soft segments. The hard segments are typically comprised of stiffer oligourethane units formed from diisocyanate and chain extender, while the soft segments are typically comprised of one or more flexible polyol units. These two types of segments will generally phase separate to form hard and soft segment domains, since they tend to be incompatible with one another. Those skilled in the relevant art, given the present teaching, will appreciate the manner in which the relative amounts of the hard and soft segments in the formed polyurethane, as well as the degree of phase segregation, can have a significant impact on the final physical and mechanical properties of the polymer. Those skilled in the art will, in turn, appreciate the manner in which such polymer compositions can be manipulated to produce cured and curing polymers with desired combination of properties within the scope of this invention.

The hard segments of the polymer can be formed by a reaction between the diisocyanate or multifunctional isocyanate and chain extender. Some examples of suitable isocyanates for preparation of the hard segment of this invention include aromatic diisocyanates and their polymeric form or mixtures of isomers or combinations thereof, such as toluene diisocyanates, naphthalene diisocyanates, phenylene diisocyanates (preferably 1,4-phenylene diisocyanate ("PPDI")), xylylene diisocyanates, and diphenylmethane diisocyanates, and other aromatic polyisocyanates known in the art. Other examples of suitable polyisocyanates for preparation of the hard segment of this invention include aliphatic and cycloaliphatic isocyanates and their polymers or mixtures or combinations thereof, such as cyclohexane diisocyanates, cyclohexyl-bis methylene diisocyanates, isophorone diisocyanates and hexamethylene diisocyanates and other aliphatic polyisocyanates. Combinations of aromatic and aliphatic or arylakyl diisocyanates can also be used.

The isocyanate component can be provided in any suitable form, examples of which include 2,4'-diphenylmethane diisocyanate, 4,4'-diphenylmethane diisocyanate, and mixtures or combinations of these isomers, optionally together with small quantities of 2,2'-diphenylmethane diisocyanate (typical of commercially available diphenylmethane diisocyanates). Other examples include aromatic polyisocyanates and their mixtures or combinations, such as are derived from phosgenation of the condensation product of aniline and formaldehyde. It is suitable to use an isocyanate that has low volatility, such as diphenylmethane diisocyanate, rather than more volatile materials such as toluene diisocyanate. An example of a particularly suitable isocyanate component is the 4,4'-diphenylmethane diisocyanate ("MDI"). Alternatively, it can be provided in liquid form as a combination of 2,2'-, 2,4'- and 4,4'- isomers of MDI. In a preferred embodiment, the isocyanate is MDI and even more preferably 4,4'-diphenylmethane diisocyanate.

Some examples of chain extenders for preparation of the hard segment of this invention include, but are not limited, to short chain diols or triols and their mixtures or combinations thereof, such as 1,4-butane diol, 2-methyl-1,3-propane diol, 1,3-propane-diol ethylene glycol, diethylene glycol, glycerol, cyclohexane dimethanol, triethanol amine, and methyldiethanol amine. Other examples of chain extenders for preparation of the hard segment of this invention include, but are not limited to, short chain diamines and their mixtures or combinations thereof, such as dianiline, toluene diamine, cyclohexyl diamine, and other short chain diamines known in the art.

The soft segment consists of urethane terminated polyol moieties, which are formed by a reaction between the polyisocyanate or diisocyanate or polymeric diisocyanate and polyol. Examples of suitable diisocyanates are denoted above. Some examples of polyols for preparation of the soft segment of this invention include but are not limited to polyalkylene oxide ethers derived form the condensation of alkylene oxides (e.g. ethylene oxide, propylene oxide, and blends thereof), as well as tetrahyrofuran based polytetramethylene ether glycols, polycaprolactone diols, polycarbonate diols and polyester diols and combinations thereof. In a preferred embodiment, the polyols are polytetrahydrofuran polyols ("PTHF"), also known as polytetramethylene oxide ("PTMO") or polytetramethylene ether glycols ("PTMEG"). Even more preferably, the use of two or more of PTMO diols with different molecular weights selected from the commercially available group consisting of 250, 650,1000, 1400, 1800, 2000 and 2900.

Two or more PTMO diols of different molecular weight can be used as a blend or separately, and in an independent fashion as between the different parts of the two part system. The solidification temperature(s) of PTMO diols is generally proportional to their molecular weights. The compatibility of the PTMO diols with such chain extenders as 1,4-butanediol is generally in the reverse proportion to molecular weight of the diol(s). Therefore the incorporation of the low molecular weight PTMO diols in the "curative" (part B) component, and higher molecular weight PTMO diols in the prepolymer (part A) component, can provide a two-part system that can be used at relatively low temperature. In turn, good compatibility of the low molecular weight PTMO diols with such chain extenders as 1,4-butanediol permits the preparation of two part systems with higher (prepolymer to curative) volume ratio. Amine terminated polyethers and/or polycarbonate-based diols can also be used for building of the soft segment.

The PU can be chemically crosslinked, e.g., by the addition of multifunctional or branched OH-terminated crosslinking agents or chain extenders, or multifunctional isocyanates. Some examples of suitable crosslinking agents include, but are not limited to, trimethylol propane ("TMP"), glycerol, hydroxyl terminated polybutadienes, hydroxyl terminated polybutadienes (HTPB), trimer alcohols, Castor oil polyethyleneoxide (PEO), polypropyleneoxide (PPO) and PEO-PPO triols. In a preferred embodiment, HTPB is used as the crosslinking agent.

This chemical crosslinking augments the physical or "virtual" crosslinking of the polymer by hard segment domains that are in the glassy state at the temperature of the application. The optimal level of chemical cross-linking improves the compression set of the material, reduces the amount of the extractable components, and improves the biodurability of the PU. This can be particularly useful in relatively soft polyurethanes, such as those suitable for the repair of damaged cartilage. Reinforcement by virtual cross-links alone may not generate sufficient strength for in *vivo* performance in certain applications. Additional cross-linking from the soft segment, potentially generated by the use of higher functional polyols can be used to provide stiffer and less elastomeric materials. In this manner a balancing of hard and soft segments, and their relative contributions to overall properties can be achieved.

Additionally, a polymer system of the present invention preferably contains at least one or more, biocompatible catalysts that can assist in controlling the curing process, including the following periods: (1) the induction period, and (2) the curing period of the biomaterial. Together these two periods, including their absolute and relative lengths, and the rate of acceleration or cure within each period, determines the cure kinetics or profile for the composition. Some examples of suitable catalysts for preparation of the formed PU of this invention include, but are not limited to, tin and tertiary amine compounds or combinations thereof such as dibutyl tin dilaurate, and tin or mixed tin catalysts including those available under the tradenames "Cotin 222", "Formrez UL-22" (Witco), "dabco" (a triethylene diamine from Sigma-Aldrich), stannous octanoate, trimethyl amine, and triethyl amine. In a preferred embodiment, the catalyst is Formrez UL-22 (Witco). In an alternative preferred embodiment, the catalyst is a combination Cotin 222 (CasChem) and dabco (Sigma-Aldrich).

Both *in vivo* and *ex vivo* cured polyurethanes for use in the present invention can be formed by the reaction of at least two parts, and optionally more parts, including for instance those providing additives and the like. Part I of which (alternatively referred to as Part A) includes a di- or multifunctional isocyanate or prepolymer which is the reaction product of one or more OH-terminated components, and one or more isocyanates, and optionally other additives such as antioxidants, acidity modifiers, and so on. Part II of the polyurethane (alternatively referred to as Part B herein) is a curative component that includes of one or more chain extenders one or more polyols, and one or more catalysts, and other additives such as antioxidants and dyes. For a suitable formed PU, the stoichiometry between Parts I (prepolymer) and II (curative component), expressed in terms of NCO:OH molar ratio of the isocyanate terminated pre-polymer (Part I) and the curative component (Part II) is preferably within the range of about 0.8 to 1.0 to 1.2 to 1.0, and more preferably from about 0.9 to 1 to about 1.1 to 1.0. In systems with more than two parts, generally the same NCO:OH ratio of the total formulation will be within the same ranges.

Optionally, a reactive polymer additive can be included and is selected from the group consisting of hydroxyl- or amine-terminated compounds selected from the group consisting of poybutadiene, polyisoprene, polyisobutylene, silicones, polyethylene-propylenediene, copolymers of butadiene with acryolnitrile, copolymers of butadiene with styrene, copolymers of isoprene with acrylonitrile, copolymers of isoprene with styrene, and mixtures of the above.
Suitable compositions for use in the present invention are those polymeric materials that provide an optimal combination of properties relating to their manufacture, application, and *in vivo* use. In the uncured state, such properties include component miscibility or compatibility, processability, and the ability to be adequately sterilized or aseptically processed and stored. In the course of applying such compositions, suitable materials exhibit an optimal combination of such properties as flowability, moldability, and *in vivo* curability. In the cured state, suitable compositions exhibit an optimal combination of such properties as strength (e.g., tensile and compressive), modulus, biocompatibility and biostability.

When cured, the compositions demonstrate an optimal combination of properties, particularly in terms of their conformational stability and retention of physical shape, dissolution stability, biocompatibility, and physical performance, as well mechanical properties such as load-bearing strength, tensile strength, shear strength, shear fatigue resistance, impact absorption, wear resistance, and surface abrasion resistance. Such performance can be evaluated using procedures commonly accepted for the evaluation of natural tissue and joints, as well as the evaluation of materials and polymers in general. In particular, a preferred composition, in its cured form, exhibits mechanical properties that approximate or exceed those of the natural tissue it is intended to provide or replace.

Fully cured polymeric (e.g., polyurethane) biomaterials suitable for use in forming components of this invention provide an optimal combination of such properties as creep and abrasion resistance. Preferably, for instance, the biomaterial provides DIN abrasion values of less than about 100 mm³, more preferably less than about 80 mm³ and most preferably less than about 60 mm³, as determined by ASTM Test Method D5963-96 ("Standard Test Method for Rubber Property Abrasion Resistance Rotary Drum Abrader").

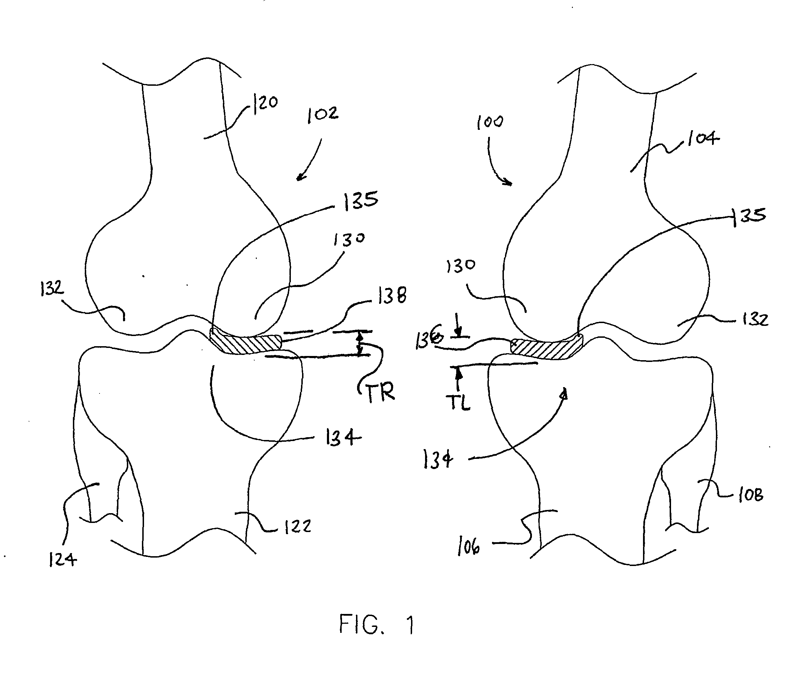

The kit of the present invention, which will typically include at least a plurality of components as described herein, will be further described with reference to the Figures, in which figure 1 is a partial front view of a human skeleton including a left leg 100 and a right leg 102. Left leg 100 includes a left femur 104, a left tibia 106 and a left fibula 108. Similarly, right leg 102 includes a right femur 120, a right tibia 122 and a right fibula 124. The patella, or knee cap, is not shown in figure 1 so that an entire right knee joint 126 and an entire left knee joint 128 are visible.

Each femur includes a medial condyle 130 and a lateral condyle 132. Each tibia includes a tibial plateau 134. In figure 1, it can be appreciated that a left implant 136 in accordance with the present invention, is interposed between the medial condyle 130 of left femur 104 and the tibial plateau 134 of left tibia 106. Similarly, a right implant 138 in accordance with the present invention is interposed between the medial condyle 130 of right femur 120 and the tibial plateau 134 of right tibia 122. In figure 1, it can be appreciated that a mesial ridge 135 of each implant extends between the medial condyle 130 and the lateral condyle 132 of a femur.

Each human knee joint includes a plurality of ligaments that extend between the femur and the tibia. In figure 1, it can be appreciated that left implant 136 and right implant 138 have a thickness TL and a thickness TR respectively. Some exemplary methods in accordance with the present invention, include the step of evaluating the laxity of the ligaments of a particular knee joint in order to determine an implant thickness suitable for that knee joint.

Because ligament laxity is likely to vary from one patient to another, certain methods in accordance with the present invention include the step of providing a stocking set of implants to a physician. This stocking set of implants can be advantageously located in the surgical suite during an operation. This stocking set can include knee implants of varying thickness to account for the ligament laxity in a particular knee joint.

Because the size of human bones (e.g., the femur and the tibia) vary from one patient to another, certain methods in accordance with the present invention include the step of providing a stocking set of implants to a physician. This stocking set can include implants of varying sizes. Some advantageous methods in accordance with the present invention include the step of measuring an extent of the tibial plateau. Some of these methods can also include the step of selecting a particular implant size base on a measured value (e.g., an extent of the tibial plateau).

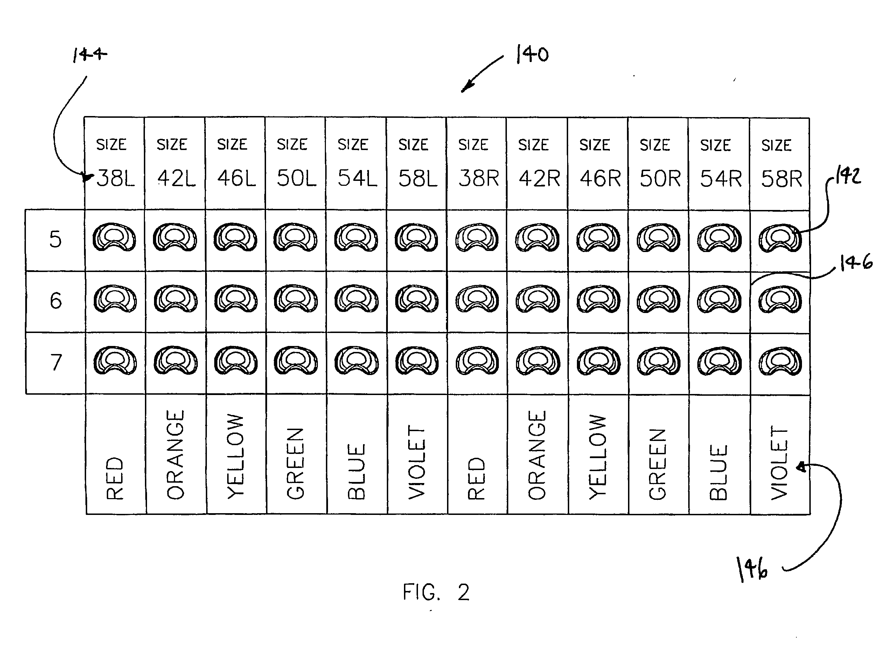

Figure 2 is a diagrammatic representation of a stocking set 140 including a plurality of implants 142. In the embodiment of figure 2, stocking set 140 includes implants 142 of varying configurations. With reference to figure 2, it will be appreciated that stocking set 140 includes left implants and right implants. In the embodiment of figure 2, implants 142 are provided in six sizes with each implant size being provided in three different thicknesses. Accordingly, a method for selecting an implant for a particular joint will typically include the step of determining an appropriate implant size and the step of determining an appropriate implant thickness. It is to be understood that various thicknesses can be utilized without deviating from the spirit and scope of the present invention. In the exemplary embodiment of figure 2, the thickness are identified with the numerals 5, 6, and 7. In the exemplary embodiment of figure 2, these numbers may correspond to thicknesses of five millimeters, six millimeters, and seven millimeters.

In the embodiment of figure 2, each size has been assigned an identifying character 144. In some embodiments of the present invention, identifying character 144 can be one or more arbitrarily selected numbers, letters or combinations of letters and numbers. In the embodiment of figure 2, one exemplary identifying character is "38L." In the exemplary embodiment of figure 2, the numeral "38" will generally correspond to a dimension of the implant. The letter L in identifying character 144 identifies those implants 142 that are intended for use with the left leg.

In the embodiment of figure 2, each implant is disposed within a box 146. In some embodiments of the present invention, each implant can be individually packaged in a sterile package represented by box 146 in figure 2. In the embodiment of figure 2, each size has been assigned an identifying characteristic 148. In the exemplary embodiment of figure 2, each identifying characteristic 148 comprises a color. In this embodiment, a portion or all of each implant having a size "42" will be orange in color. In some methods in accordance with the present invention, a plurality of trial implants corresponding to each implant 142 are provided to a surgeon. Methods in accordance with the present invention are possible in which each trial implant has a color that substantially matches the color of a corresponding implant.

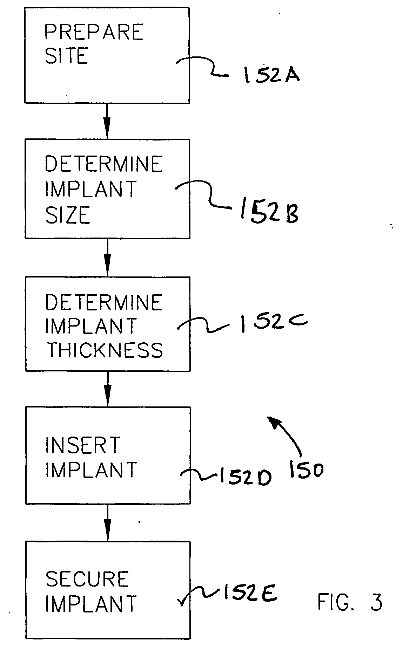

Figure 3 is a flow chart 150 illustrating a method in accordance with an exemplary embodiment of the present invention. The flow chart of figure 3 provides a general overview of an exemplary method in accordance with the present invention. Methods and apparatus in accordance with the present invention will also be discussed in greater detail below.

Block 152a in figure 3 represents the step of preparing the site. In some exemplary methods in accordance with the present invention, the step of preparing the site includes the step of separating osteophytes from the tibia using a tool configured especially for that purpose. The step of preparing the site can also include the use of a tibial smoothing tool and/or a femural smoothing tool. These tools can be used to provide smooth femoral condyles and a smooth tibial plateau.

Block 152b illustrates the step of determining a desire implant size. The step of determining the implant size can include the step of measuring one or more dimensions of a j oint. For example, a caliper in accordance with the present invention can be used to measure the width and/or the depth of a tibial plateau.

Block 152c illustrates the step of determining an implant thickness. One goal of this step is to determine an implant thickness that will provide a full range of motion, without over compensating for ligament laxity. A kit in accordance with the present invention can include a plurality of implant templates of varying thickness as trial devices. The implant templates, can have geometry that is similar to a knee implant in accordance with the present invention, except that the posterior lip can be about half to one third as deep in order to assist the surgeon in insertion and removal of the spacers. Additionally, each implant template can advantageously include a handle fixed to the body of the spacer.

Block 152d represents the step of inserting the appropriate implant. A gripping tool in accordance with the present invention can be used to facilitate holding of the implant while it is inserted into a joint.

Block 152e represents the step of securing the implant. An implant in accordance with the present invention can include tabs, sutures, and the like to facilitate securing of the implant. For example, sutures can be molded into the implant and extend away from the implant. Optionally, sutures may be added by the surgeon or others, for instance, by the use of preformed holes or tabs within or upon the implant itself. These sutures can be attached to the body of a patient in order to secure the implant. Some or all of the steps illustrated in flow chart 150 can be conducted in conjunction with arthroscopic visualization. For example, arthroscopic visualization can be used to read a measurement from a measuring device.

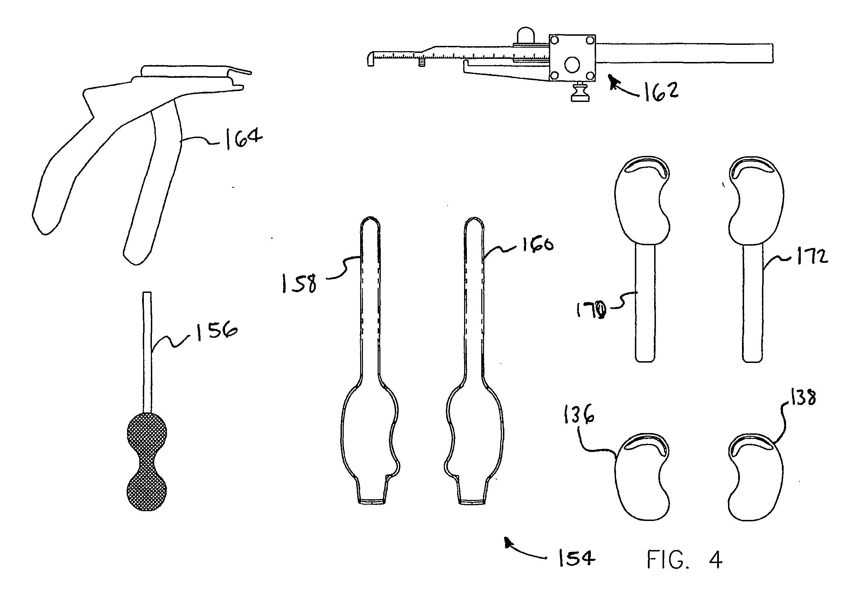

Figure 4 is a plan view of a kit 154 in accordance with an exemplary embodiment of the present invention. Kit 154 of figure 4 includes three site preparation tools that can be used for preparing a joint to receive an implant. These site preparation tools include a tibial prep tool 156, a left femoral prep tool 158 and a right femoral prep tool 160. Left femoral prep tool 158 and right femoral prep tool 160 can be used, for example, to remove non-bone material from the bone of a femoral condyle to provide a smooth surface. Tibial prep tool 156 can be used, for removing non-bone material from the bone of the tibial plateau.

Kit 154 also includes a measuring device 162 that can be used for determining an appropriate implant size. In some advantageous embodiments of the present invention, measuring device 162 is configured for measuring one or more dimensions of a tibial plateau.

Kit 154 of figure 4 includes a left implant 136, a right implant 138 and a gripper 164 that can facilitate insertion of each implant into a joint. Although one left implant 136 and one right implant 138 are shown in the exemplary embodiment of figure 4, certain advantageous methods in accordance with the present invention include the step of providing a plurality of left implants and a plurality of right implants. For example, a stocking set of left implants and a stocking set of right can be provided. This stocking set can include implants in a variety of sizes and thicknesses.

Kit 154 of figure 4 also includes a right implant template 172 and a left implant template 170. In some methods in accordance with the present invention, a plurality of left implant templates 170 and right implant templates 172 are provided for determining an appropriate implant thickness. Although one left implant template 170 is shown in the exemplary embodiment of figure 4, certain advantageous methods in accordance with the present invention include the step of providing as plurality of implant template kits with each kit corresponding to a particular size of implant. In certain advantageous embodiments of the present invention, each implant template kit comprises a plurality of implant templates of varying thickness.

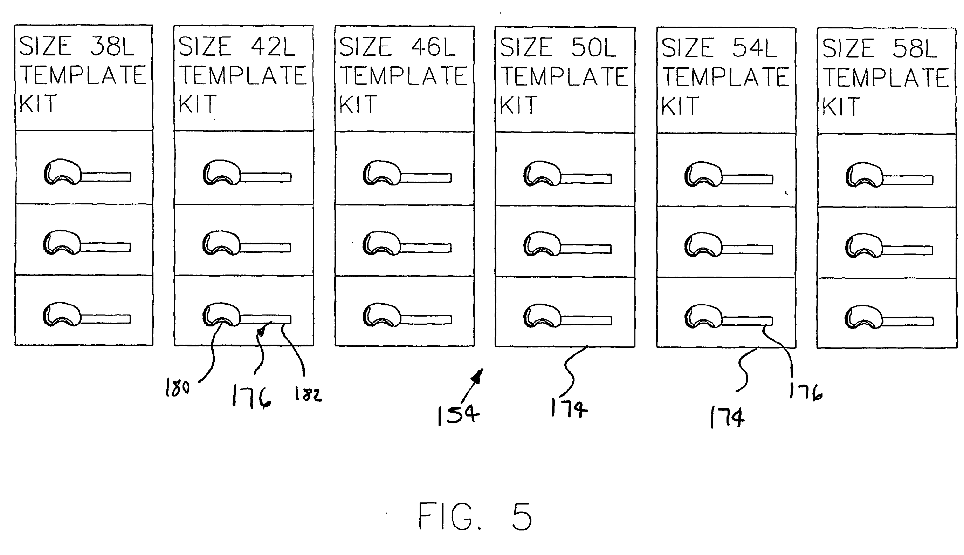

Figure 5 is a diagrammatic illustration of an evaluation kit 154 comprising a plurality of implant template kits 174. With reference to figure 5, it will be appreciated that each implant template kit corresponds to an implant size. In the embodiment of figure 5, each implant template kit 154 includes implant templates 176 of varying thicknesses. Once a desire implant size has been determined using a measuring step, the corresponding implant template kit 154 can be selected. In the embodiment of figure 5, each implant template 178 comprises a body 180 and a handle 182. In some embodiments of the present invention, the body 180 of each implant template has width and length dimensions that are substantially similar to a corresponding implant. In some advantageous embodiments, the lateral lip portion of each implant template is generally lower than that of a corresponding implant to aid in inserting and removing the implant templates. The handle 182 of each implant template 178 also aids in inserting and removing the implant template 178. A plurality of implant templates can be enclosed in a single sterile package.

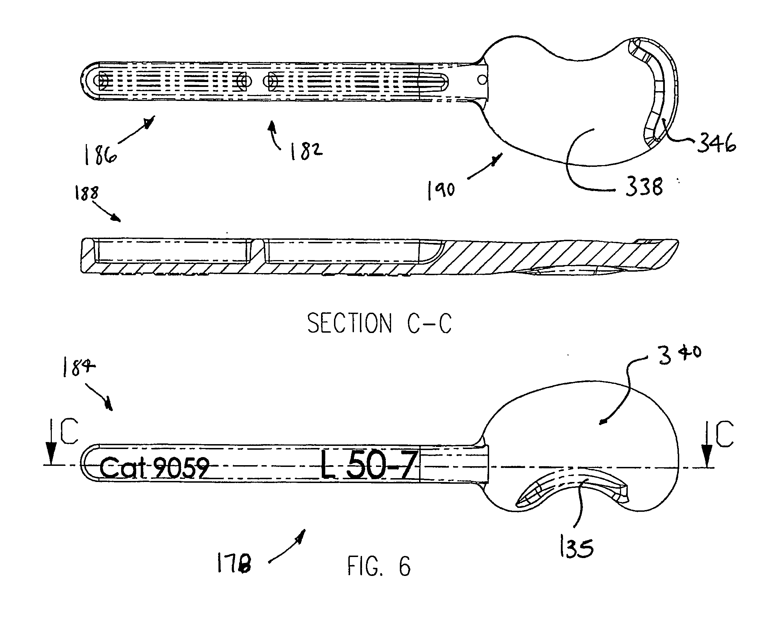

Figure 6 includes a number of views showing an implant template 178 in accordance with the present invention. More particularly, figure 6 includes a top view 184, a bottom view 186 and a cross-sectional side view 188. With reference to figure 6, it will be appreciated the implant template 178 comprises an implant-like portion 190 and a handle 182. Implant-like portion 190 comprises a first major surface 338 adapted to be positioned upon the tibial plateau of a tibia, and a second major surface 340 adapted to be positioned against the medial condyle of a femur. As shown in figure 6, implant-like portion 190 also comprises a tibial projection 346 extending beyond first major surface 338 and mesial ridge 135 extending beyond second major surface 340.

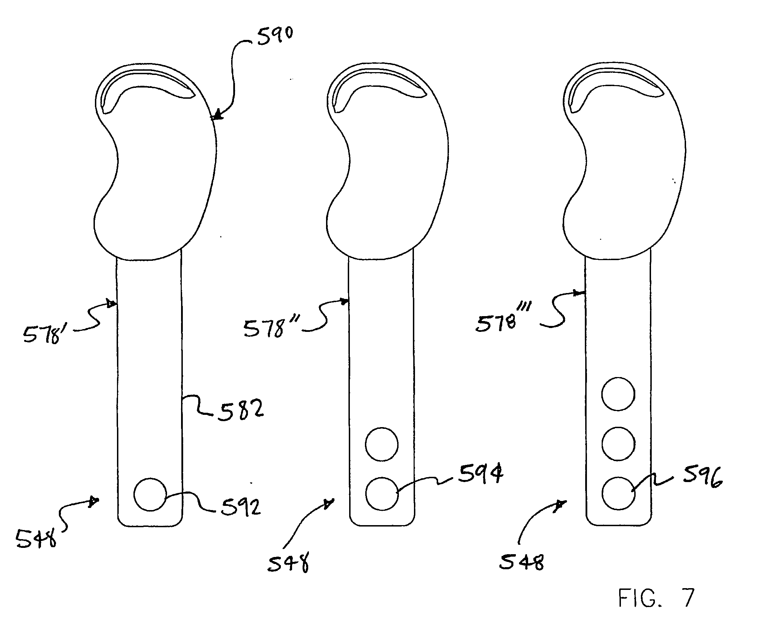

Figure 7 is a plan view showing a first implant template 578', a second implant template 578", and a third implant template 578"'. Each implant template includes an implant-like portion 590 and a handle 582. In the embodiment of figure 7, each implant template also includes an identifying characteristic 548. The identifying characteristic 548 of first implant template 578', for example, comprises a single hole 592 defined by handle 582 of first implant template. As shown in figure 7, the identifying characteristic 548 of second implant template 578" comprises two holes 594 defined by handle 582 of second implant template, whereas the identifying characteristic 548 of third implant template 578"' comprises three holes 596 defined by handle 582 of third implant template. Optionally, or in addition, templates can be molded in colors to duplicate the corresponding color of the actual implant.

In some embodiments of the present invention, the identifying characteristic 548 of each implant template can correspond to a thickeness of the implant template. For example, implant templates having one, two and three holes can be provided in thicknesses of five millimeters, six millimeters, and seven millimeters respectively. In some embodiments of the present invention, each implant template is fabricated by injection molding and the identifying characteristic 548 of each implant template is created during that injection molding process. Molding in the identifying characteristic can reduce the likelihood that an implant template is mis-labeled with an incorrect identifying characteristic.

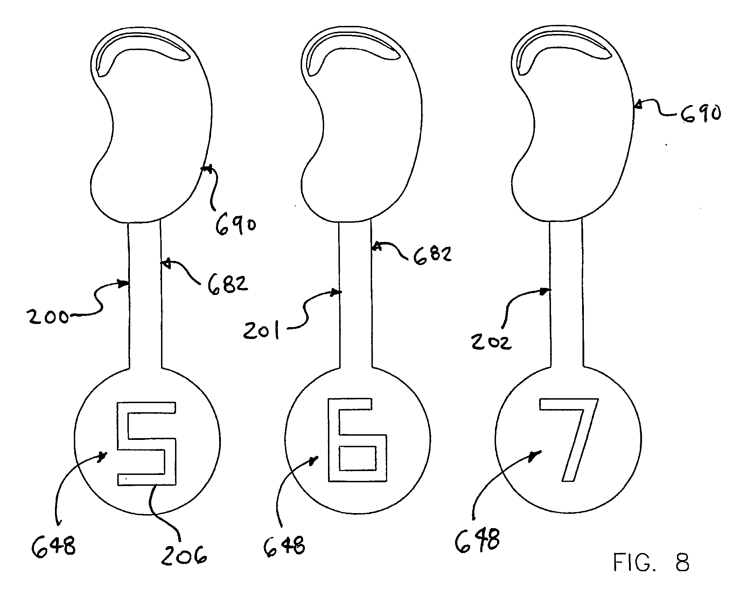

Figure 8 is a plan view showing a first trial 200, a second trial 201, and a third trial 202. Each trial includes a implant-like portion 690 and a handle 682. In the embodiment of figure 8, each trial 204 also includes an identifying characteristic 648. The identifying characteristic 648 of first trial 200, for example, comprises a number five 206 defined by handle 682 of first trial 200. In the exemplary embodiment of the figure 8, this number five indicates that first implant template has a thickness of about five millimeters. As shown in figure 8, the identifying characteristic 648 of second trial 201 comprise a number six that is defined by handle 682 of second trial, whereas the identifying characteristic 648 of third trial 202 comprises a number seven defined by handle 682 of third trial. In the exemplary embodiment of figure 8, the number six defined by handle 682 of second trial 201 can indicate that second trial 201 has a thickness of about six millimeters. Also in the exemplary embodiment of figure 8, the number seven defined by handle 682 of third trial 202 can indicate that third trial 202 has a thickness of about seven millimeters.

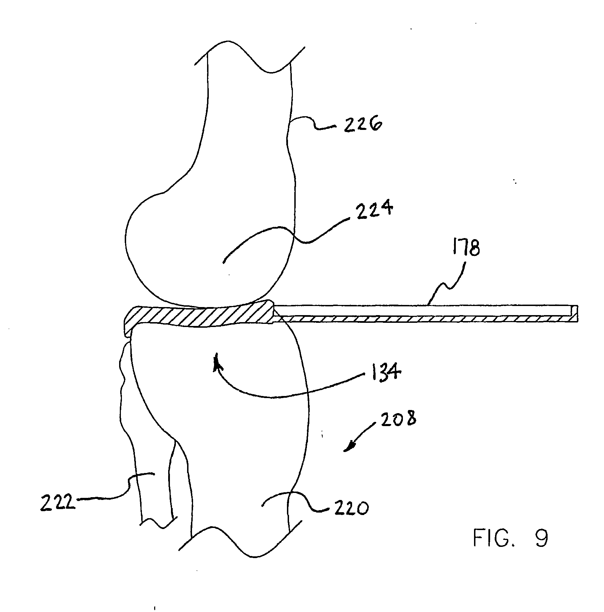

Figure 9 is a side view including an implant template 178 shown in cross-section. A lower leg 208 including a tibia 220 and a fibula 222 is also shown in figure 9. In the embodiment of figure 9, implant template 178 is interposed between a medial condyle 224 of a femur 226 and a tibial plateau 134 of tibia 220.

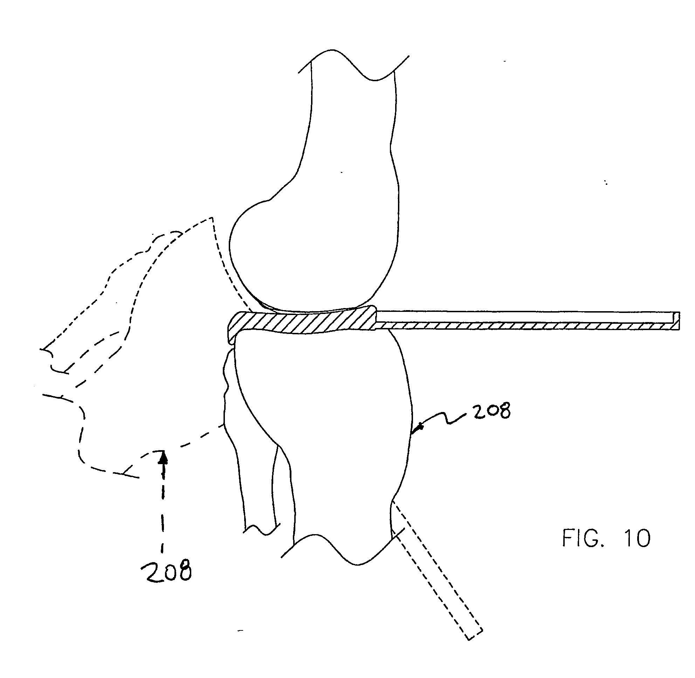

Figure 10 is an additional side view illustrating a sizing method in accordance with the present invention. In the embodiment of figure 10, lower leg 208 is disposed in a first position. A second position of lower leg 208 is illustrated using dashed lines in figure 10. In some methods in accordance with the present invention, a implant template is inserted into the knee joint, and the lower leg 208 is put through a range of motion. While the lower leg is moved, a physician can evaluate the knee for proper ligament tension. The physician can also check that the leg is capable of covering an appropriate range of motion. An implant template 178 is visible in figure 10. Implant template 178 includes an implant-like portion 190 and a handle 182.

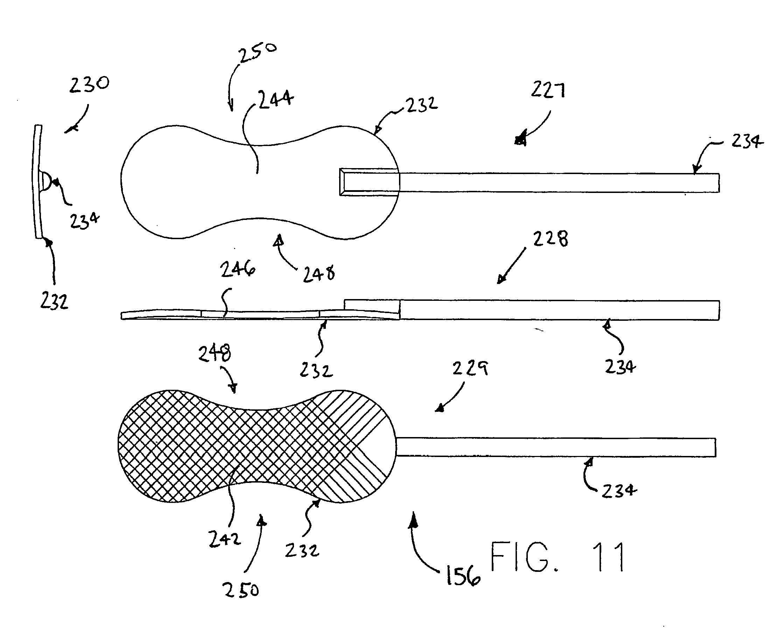

Figure 11 includes a number of views showing a tibial prep tool 156 in accordance with an exemplary embodiment of the present invention. More particularly, figure 11 includes a top view 227, a side view 228, a bottom view 229 and an end view 230. Tibial prep tool 156 of figure 11 includes a body portion 232 and a shaft 234. Body portion 232 of tibial prep tool 156 comprises a head 240 having a bottom surface 242, a top surface 244, and a side surface 246. In the embodiment of figure 11, top surface 244 of head 240 is relatively smooth when compared with bottom surface 242.

The step of preparing a site to receive an implant can include the use of tibial prep tool 156 to proved a smooth bone surface. The tool can be held in position on a portion of the tibial plateau and reciprocated, for example, using a suitable power instrument or be manipulated by hand. The smoothing device can be secured to a powered driver (e.g., a Triton brand reciprocating saw) by inserting shaft 234 and tightening the collett of the driver to grasp the shaft. The speed of the driver can be controlled in two ways, namely, by either limiting the air pressure delivered to the driver using an air regulator, and/or by a variable speed valve on the driver, which provides more speed (strokes per second) with increased depression of the control lever. Tibial prep tool 156 can be manipulated around and within a joint space. Tibial prep tool 156 can be guided, for example, by placing an index finger on top surface 244 of body portion 232.

In the embodiment of figure 11, body portion 232 defines a first cut-out 248 and a second cut-out 250. One cut-out can be dimensioned to receive the intercondylar eminence (ICE) of a left leg and the other cut-out can be dimensioned to receive the intercondylar eminence (ICE) of a left leg. Accordingly, the presence of first cut-out 248 and second cut-out 250 can allow tibial prep tool 156 to be used with both a left leg and a right leg.

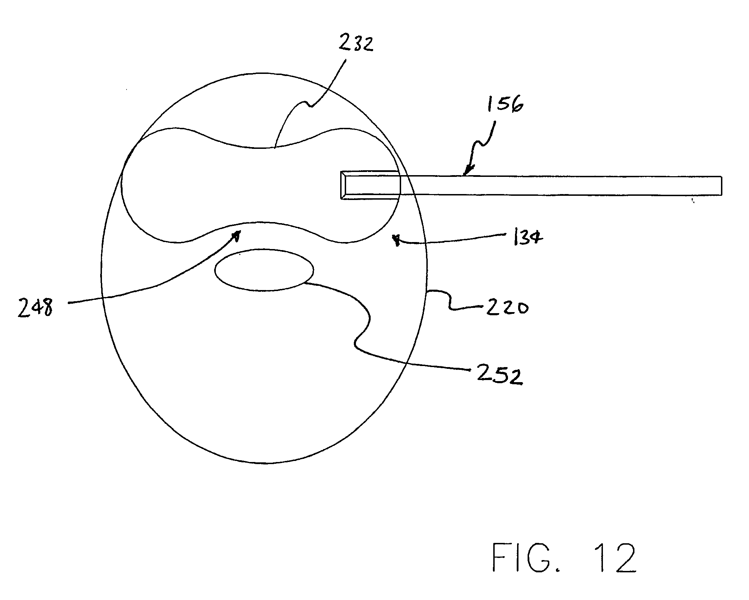

Figure 12 is a top view showing tibial prep tool 156 disposed proximate a tibial plateau 134 of a tibia 220. With reference to figure 12, it will be appreciated that first cut-out 248 of body portion 232 of tibial prep tool 156 is dimensioned to receive the intercondylar eminence (ICE) 252 of tibia 220.

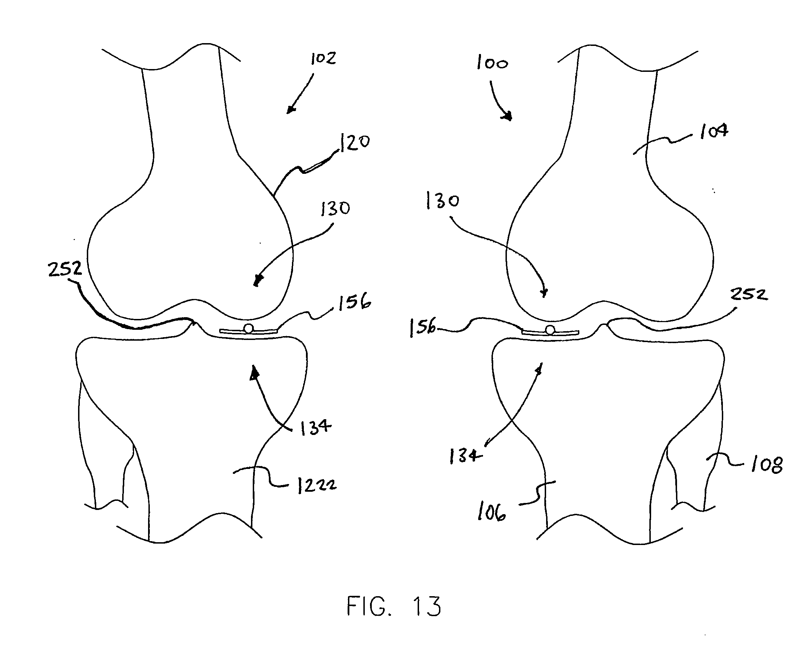

Figure 13 is a partial front view of a human skeleton including a left leg 100 and a right leg 102. Left leg 100 includes a left femur 104, a left tibia 106 and a left fibula 108. In figure 13, tibial prep tool 156 is shown disposed between a tibial plateau 134 of left tibia 106 and a medial condyle 130 of left femur 104. With reference to figure 13, it will be appreciated that tibial prep tool can be used in conjunction with either left leg 100 or right leg 102. In figure 13, tibial prep tool 156 is shown disposed above a tibial plateau 134 of a right tibia 122 of right leg 102. With reference to figure 13, it will be appreciated that this tibial prep tool 156 is also located below a medial condyle 130 of a right femur 120 of right leg 102. An intercondylar eminence (ICE) 252 of each tibia is also visible in figure 13.

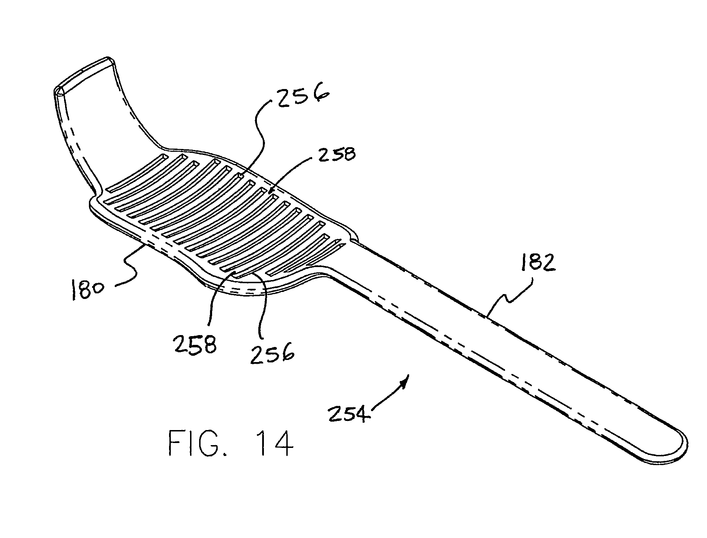

Figure 14 is a perspective view of a femoral prep tool 254 in accordance with an exemplary embodiment of the present invention. Femoral prep tool 254 includes a body 180 and a handle 182. With reference to figure 14, it will be appreciated that body 180 defines a plurality of grooves 256. A ridge 258 is disposed between each groove 260. These ridges and grooves can be used to, for example, to remove non-bone material from the bone of a femoral condyle to provide a smooth surface.

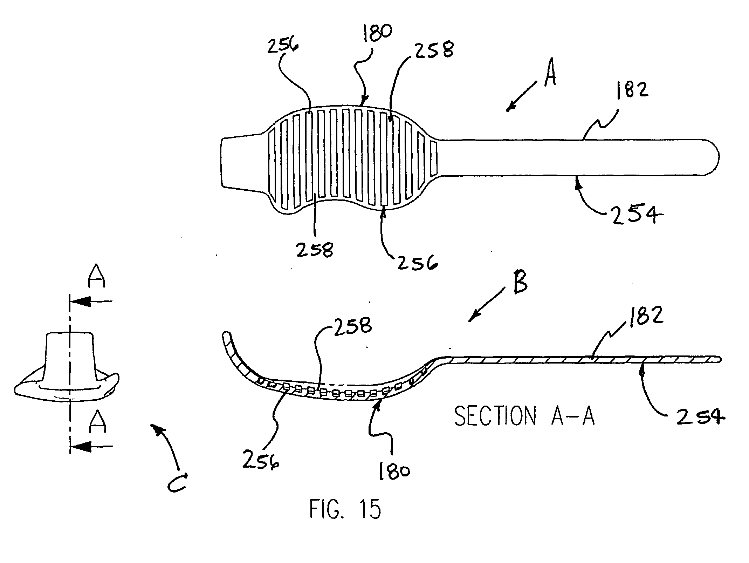

Figure 15 includes a number of views showing a femoral prep tool 254 in accordance with an exemplary embodiment of the present invention. More particularly, figure 15 includes a top view 184, a cross sectional side view 228 and an end view 230. In one exemplary smoothing method of the present invention, a femoral prep tool 254 is positioned between the femoral condyles and the tibial plateau. Force is then applied to the lower leg so as to press the femoral prep tool against the femoral condyles. The knee is put through a range of motion while the femoral prep tool is pressed against the femoral condyles. The femoral condyle is periodically evaluated for smoothness, for example, by finger palpitation. If there are ridges that can be abrasive to an implant, additional material removal can be required. The femoral prep tool can be used to, for example, to remove non-bone material from the bone of a femoral condyle to provide a smooth surface. Femoral prep tool 254 of figure 15 includes a body 180 and a handle 182. With reference to figure 15, it will be appreciated that body 180 defines a plurality of grooves 256. A ridge 258 is disposed between each groove 260.

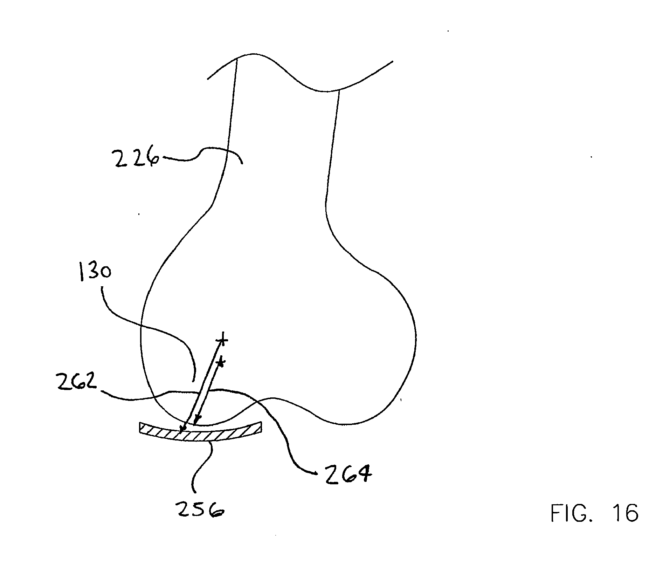

Figure 16 is an elevation view in which a femoral prep tool 254 is shown in lateral cross-section. In the embodiment of figure 16, femoral prep tool 254 is disposed proximate a medial condyle 130 of a femur 226. With reference to figure 16, it will be appreciated that femoral prep tool 254 has a lateral radius 262 that is similar to a first radius 264 of medial condyle 130. More particularly, in the exemplary embodiment of figure 16, lateral radius 262 is slightly larger than first radius 264 of medial condyle 130.

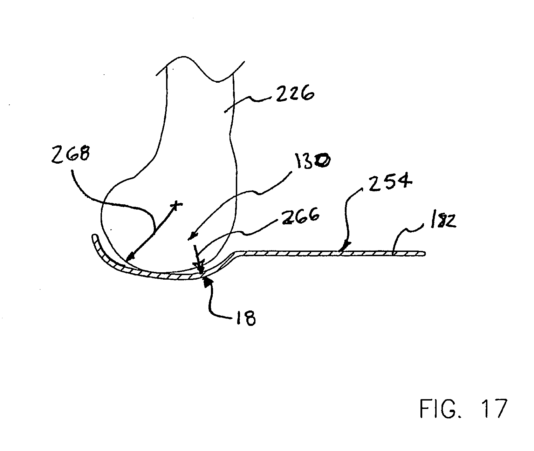

Figure 17 is a side view illustrating femoral prep tool 254 shown in the previous figure. A handle 182 of femoral prep tool 254 is visible in figure 17. With reference to figure 17, it will be appreciated that femoral prep tool 254 has a longitudinal radius 266 that is similar to a second radius 268 of medial condyle 130.

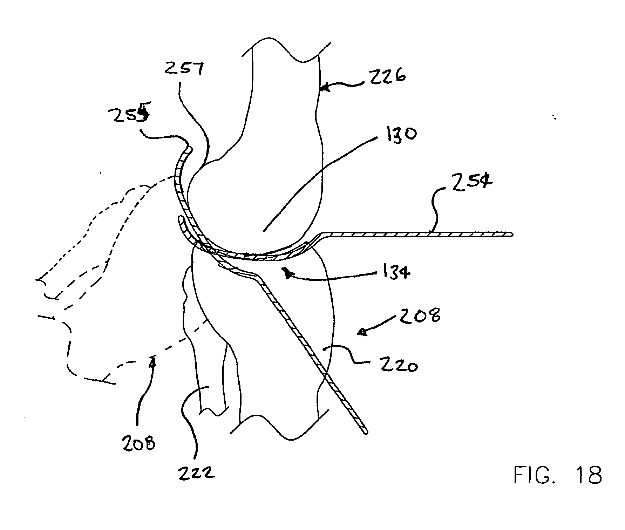

Figure 18 is an additional side view illustrating an exemplary smoothing step of an exemplary method in accordance with the present invention. A lower leg 208 including a tibia 220 and a fibula 222 is shown in figure 18. In the embodiment of figure 18, femoral prep tool 254 is shown interposed between a tibial plateau 134 of tibia 220 and a medial condyle 130 of femur 226. In some methods in accordance with the present invention, force applied to lower leg 208 can be used to press femoral prep tool 254 against medial condyle 130 of femur 226.

In the embodiment of figure 18, lower leg 208 is disposed in a first position. A second position of lower leg 208 is illustrated using dashed lines in figure 18. In some methods in accordance with the present invention, lower leg 208 is put through a range of motion so that femoral prep tool 254 smooths medial condyle 130. This exemplary method may, for example, remove non-bone material from the bone of a femoral condyle to provide a smooth surface. Medial condyle 130 can be periodically evaluated for smoothness, for example, by finger palpitation. If there are ridges that can be abrasive to an implant, additional material removal can be required.

In certain advantageous methods, any osteophytes that may impinge on an implant or limit range of motion are removed prior to insertion of the implant. In certain advantageous embodiments of the present invention, a distal tip 255 of femoral prep tool 254 is configured for removing osteophytes from a posterior surface 257 of the femur.

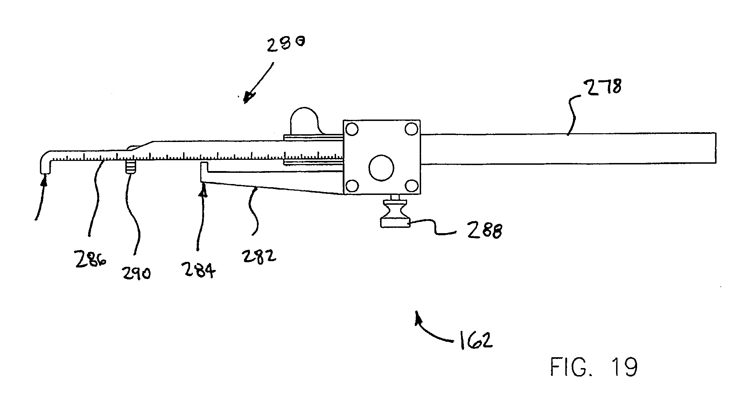

Figure 19 is a front view of a measuring device 162 in accordance with the present invention. Measuring device 162 of figure 19 includes a handle assembly 278 and gauge portion 280. The distal end of gauge portion 280 is preferably adapted to engage (e.g., hook over) the posterior edge of the tibial plateau. The gauge is sized so that it can be positioned without interference from the femoral condyle. A slide 282 having raised contact end portion 284, which translates back and forth relative to rule 286 can be positioned against the anterior portion of the tibia. A locking screw 288 is provided for selectively precluding relative motion between slide 282 and rule 286..

Measuring device 162 of figure 19, also includes a probe 290 that can be positioned along the length of rule 286, and optionally moved laterally thereto, in order to measure the depth of any indentation, or bowl shape that the tibial surface may have. Preferably, probe 290 is mounted on a slide, moveable longitudinally with the axis of rule 286, to permit it to be adjusted to make depth measurements in various locations. These dimensions provide for characterization of the tibial surface and allow for proper sizing of an implant.

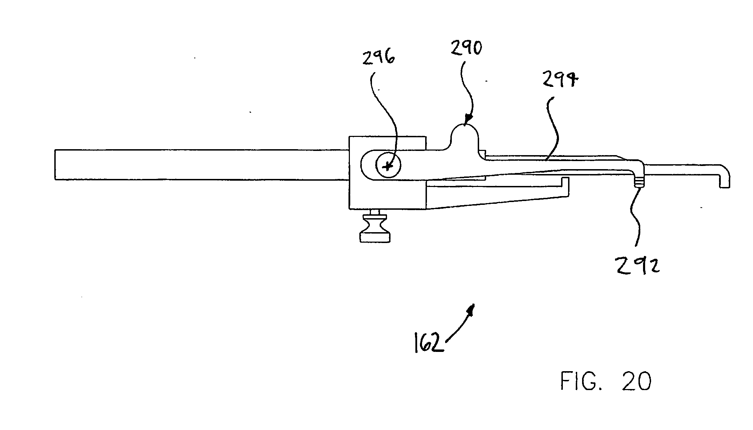

Figure 20 is a rear view of measuring device 162 shown in the previous figure. In figure 20, it can be appreciated that probe 290 comprises a tip portion 292 and an arm portion 294. In the embodiment of figure 20, probe 290 pivots about a pivot axis 296.

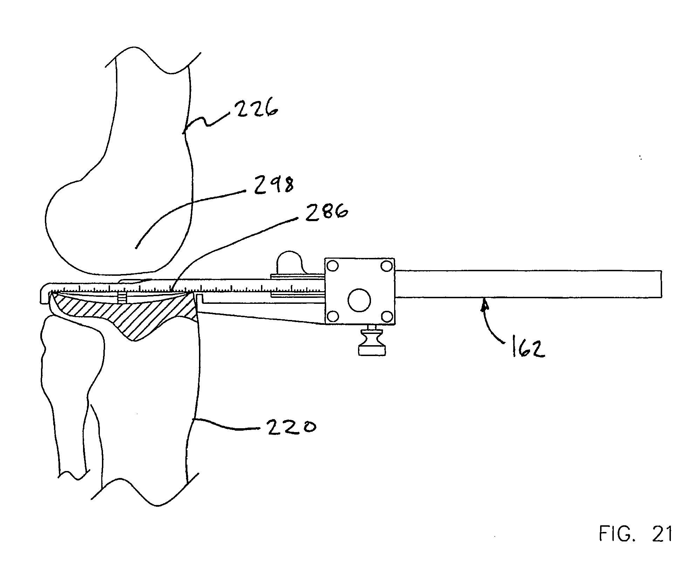

Figure 21 is a side view illustrating a measuring method. In figure 21, measuring device 162 is interposed between a condyle 298 of a femur 226 and a tibial plateau of a tibia 220. In some advantageous embodiments of the present invention, measuring device 162 is sized so that it can extend through the space between a femoral condyle and a tibia without substantially interfering with the femoral condyle and the tibia. An anterior-posterior dimension can be read from rule 286 as the distance between the point contacting the posterior tibial surface edge and a point contacting the anterior edge.

An exemplary method of measuring an extent of a tibial plateau and the depth of a tibial dish, can comprise the following steps:
1. Insert the device into the knee through arthrotomy.
2. Hook posterior edge of tibia with a distal point (e.g., tab) associated with the ruler.
3. Slide caliper mechanism until it contacts the anterior edge of the tibia.
4. Secure the anterior contact point (e.g., using a lock-screw) and removing the device from the knee.
5. Read distance off ruler.
6. Loosen screw on depth gauge dove-tail.
7. Adjust the depth gauge so that the measuring tab is centered between surfaces of caliper.
8. Insert back into the knee with the anterior and posterior contact points returned to their positions.
9. Move measuring tab of the depth gauge until it contacts tibial surface.
10. Tighten the depth gauge lock-screw and remove from knee.
11. Read the depth off the ruler.

The device and method illustrated in figure 21 is particularly adapted for use in determining an optimal size for an implant to be inserted into the joint. In a particularly preferred embodiment, as described below, the implant is designed to provide a glide path with respect to the femoral condyle. Such a device can be used in patients having joints that have progressed to the stage of "bone on bone", and thus provides a replacement for the function of articular cartilage as well as meniscus, and particularly at the central weight-bearing area, in order to restore alignment, providing an elastomeric, cushioning function. In turn, the present implant is more permanently anchored in place, in significant part by one or more posterior projections, such as the posterior lip, as well by the optional but preferred use of anterior fixation means (such as embedded sutures).

Once positioned, the depth gauge slide can be moved to a desired point along the axis of the device in order to position the depth probe in a desired location, generally to determine a maximum depth of the tibial surface. The probe can be positioned in any suitable fashion, e.g., as a set distance from either the posterior or anterior caliper, or at a midpoint between the two. For the most part, the midpoint of the tibial plateau is going to be very close to the maximum depth of the concavity. Optionally, the probe or other suitable means can also be used to scan or probe the tibial surface in order to identify the particular point of maximum depth.

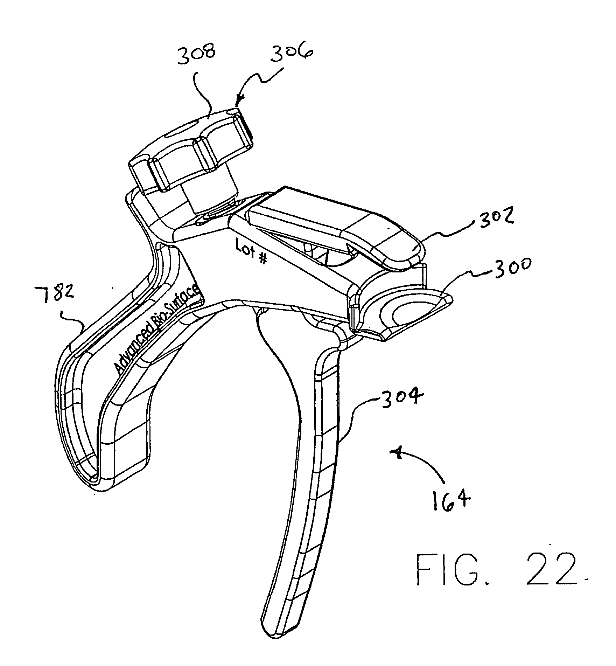

Figure 22 is a perspective view of a gripper 164 in accordance with an exemplary embodiment of the present invention. Gripper 164 of figure 22 comprises a first jaw 300 and a second jaw 302. Gripper 164 can facilitate the insertion of an implant into a joint by providing a means for firmly holding the implant. With reference to figure 22, it can be appreciated that gripper 164 comprises a handle 782 and a hand grip 304. Gripper 164 also includes a lock mechanism 306 that can that can be used to lock an implant between first jaw 300 and second jaw 302. In the embodiment of figure 22, lock mechanism 306 comprises a knob 308. The orientation and configuration of the jaws, and in turn, the manner in which they cooperate to grip an implant, are preferably designed to provide an optimal combination of gripping power sufficient for to position the implant, while providing little or no damage to the implant itself. Applicants have discovered the manner in which the configuration of a preferred implant itself provides aspects, in terms of raised and thicker portions, that facilitate the use of gripper jaws having a corresponding shape and dimension.

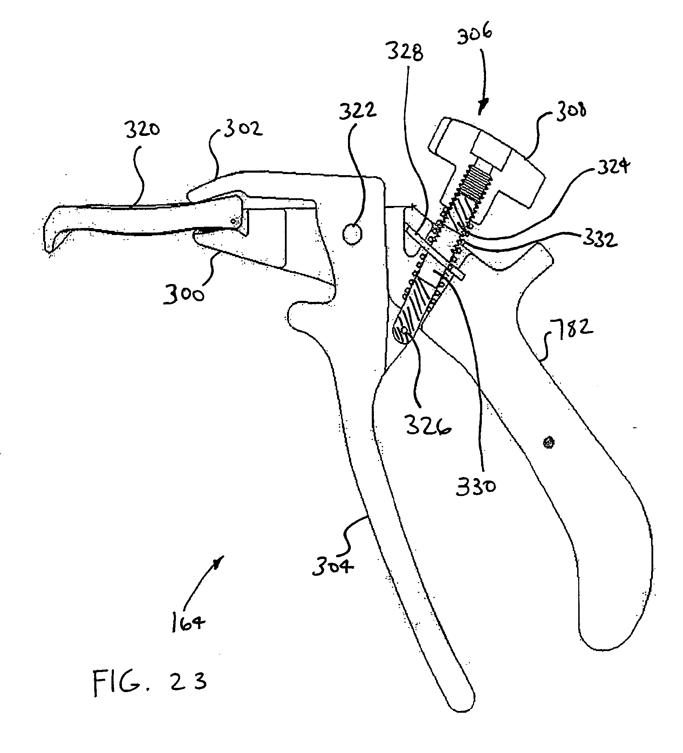

Figure 23 is a cross-sectional view of gripper 164 shown in the previous figure. An implant 320 is held between first jaw 300 and second jaw 302 of gripper 164. With reference to figure 23, it can be appreciated that hand grip 304 and second jaw 302 are pivotally coupled to handle 782 and first jaw 300 by a first dowel 322. Lock mechanism 306 comprises a bolt 324 that is pivotally coupled to hand grip 304 by a second dowel 326. Other optional lock mechanisms including ratchet and friction type catches. In the embodiment of figure 23, bolt 324 is captured relative to handle 782 by a third dowel 328 which extends through an aperture 330 defined by bolt 324. In the embodiment of figure 23, a knob 308 and a spring 332 are disposed about bolt 324. Spring 332 can act to bias hand grip 304 toward an extended position relative to handle 782.

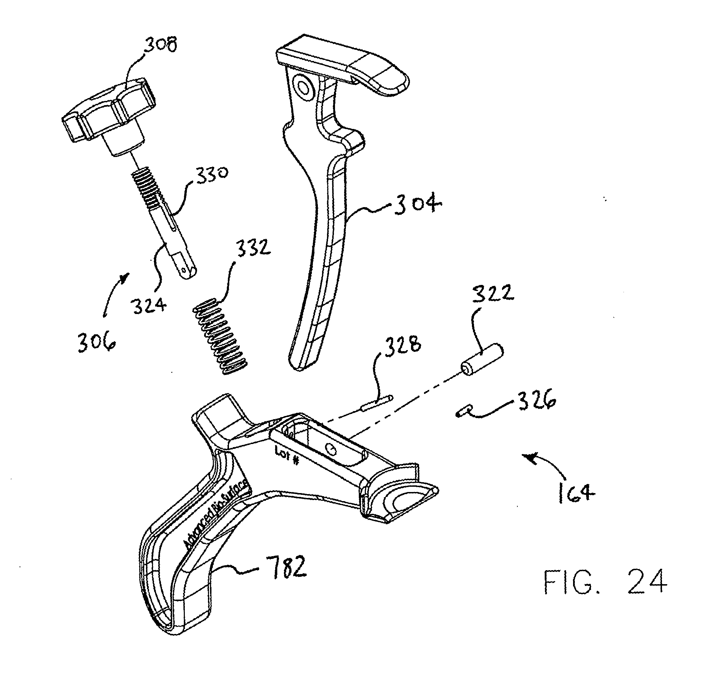

Figure 24 is an exploded view of gripper 164 shown in the previous figure. With reference to figure 24, it can be appreciated that lock mechanism 306 comprises a spring 332, a bolt 324 and a knob 308. An aperture 330 defined by bolt 324 is visible in figure 24. In the embodiment of figure 24, aperture 330 is dimensioned so as to allow a third pin 328 to extend through bolt 324. A second dowel 326 can be used to pivotally couple bolt 324 to hand grip 304. Hand grip 304 can be pivotally coupled to handle 782 by a first dowel 322.

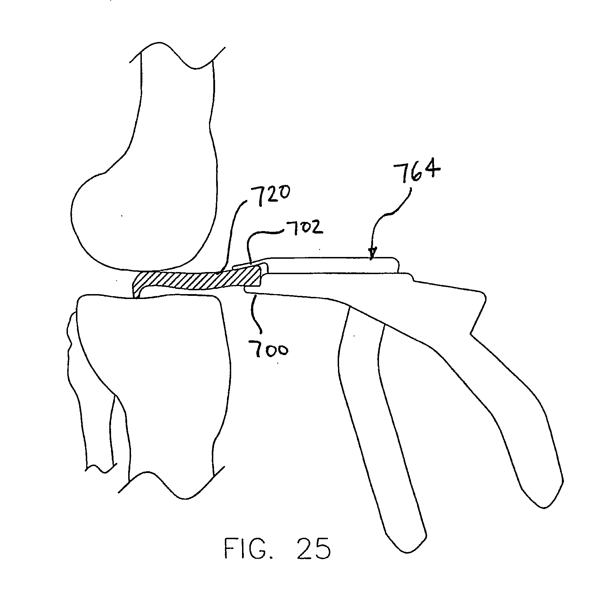

Figure 25 is a side view showing a leg and a gripper 764. An implant 720 is held between a first jaw 700 and a second jaw 702 of gripper 764. In some exemplary embodiments of the present invention, a gripper us used to hold an anterior portion of an implant while a posterior portion of the implant is inserted between a medial condyle of a femur and tibial plateau of a tibia.

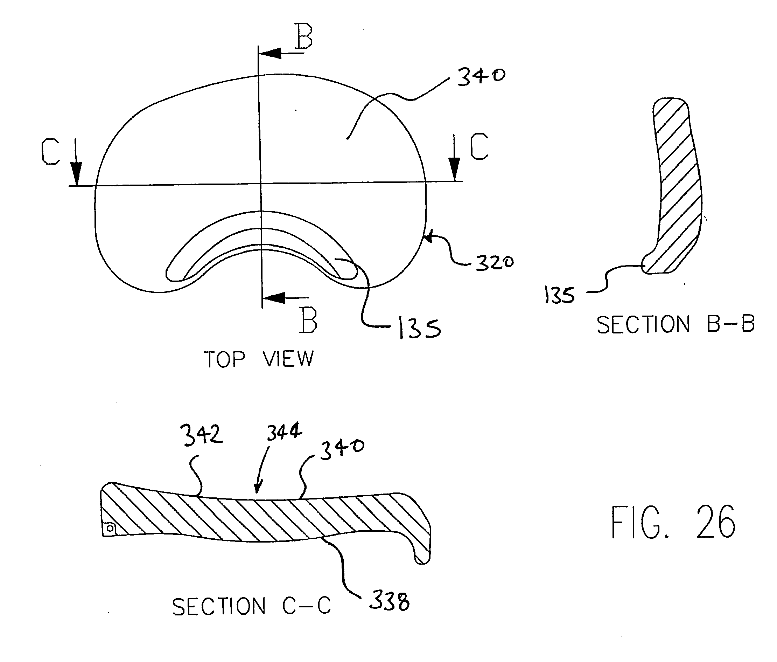

Figure 26 shows various views of an implant 320 in accordance with an additional exemplary embodiment of the present invention. The views of figure 26 include a top view, a section view (B-B) taken along section line B-B of the top view and a section view (C-C) taken along section line C-C of view (a). Implant 320 comprises a first major surface 338 adapted to be positioned upon the tibial plateau of a tibia, and a second major surface 340 adapted to be positioned against the medial condyle of a femur. Second major surface 340 preferably provides a femoral glide path 342 to facilitate its performance *in situ*, in the form of a generally central depression 344. As shown in figure 26, implant 320 also comprises a mesial ridge 135 extending beyond second major surface 340. In some advantageous embodiments of the present invention, mesial ridge 135 is dimensioned so as to extend between the medial condyle and the lateral condyle of a femur.

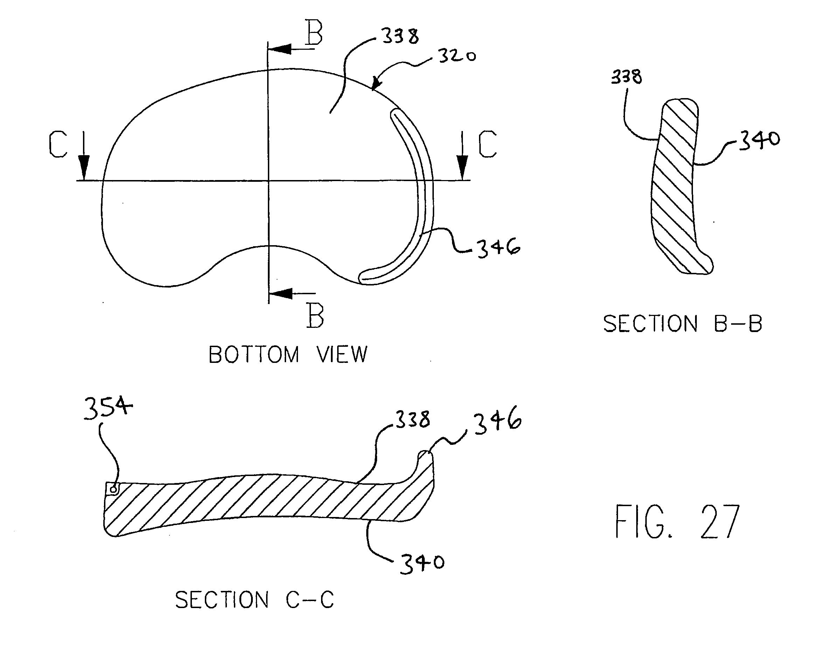

Figure 27 shows various views of implant 320 shown in the previous figure. The views of figure 27 include a bottom view, a section view (B-B) taken along section line B-B of the bottom view and a section view (C-C) taken along section line C-C of view (a). Implant 320 comprises a first major surface 338 adapted to be positioned upon the tibial plateau of a tibia, and a second major surface 340 adapted to be positioned against the medial condyle of a femur.

As shown in figure 27, implant 320 comprises a tibial projection 346 extending beyond first major surface 338. In some advantageous implementations of the present invention, tibial projection 346 is adapted to catch a posterior portion of the tibial plateau by extending over the rim of the tibial plateau. Fixation of implant 320 in situ can be accomplished by effectively capping the tibial plateau with tibial projection 346 extending distally over the rim of the plateau at one end of implant 320 and attaching another end of implant 320 with sutures. Implant 320 of figure 27 defines a hole 354. In some embodiments of the present invention, hole 354 is dimensioned so as to allow one or more sutures to pass through implant 320. The first major surface 338 of implant 320 provides with a convex bottom configuration in order to better conform to the cavity of an arthritic posterior tibial plateau.

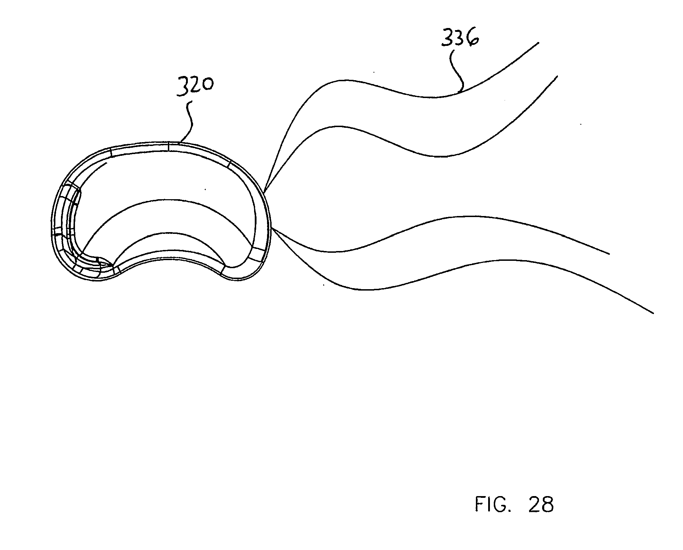

Figure 28 is a top view showing an implant 320 including a plurality of tethers 336. Tethers 336 can comprise, for example, sutures or fibrous materials that are incorporated into or onto the material of implant 320, for use in improving the initial and/or long term retention of implant 320 *in situ*, e.g., by tethering implant 320 in a desired position proximate a joint. Such other materials can be temporarily positioned into or upon a mold during molding of an implant so that they become integrated into the material of the implant as that material fills the mold. With the resulting component positioned *in situ,* tethers 336 can be used to tether the implant, by securing them to the surrounding soft tissue and/or bone by use of adhesives, sutures, screws, pins, staples, or the like, and other types of anchors, or combinations thereof, which in turn can be prepared using bioabsorbable and/or non-bioabsorbable cements, composites, and adhesives. The tethers can provide both an immediate fixation function, and optionally also a desired long term function, by permitting them to be either absorbed by the body over time, and/or to permit or encourage fibrous tissue ingrowth for long term fixation.

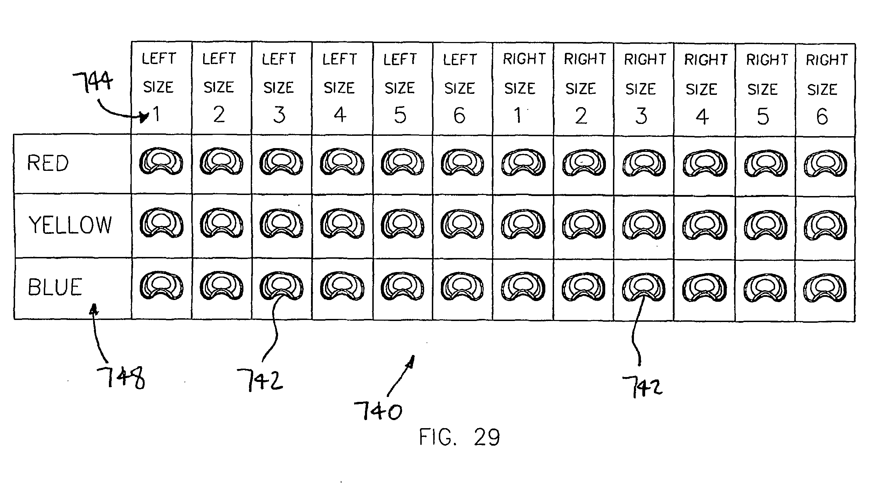

Figure 29 is a diagrammatic representation of a stocking set 740 including a plurality of implants 742. In the embodiment of figure 29, stocking set 740 includes 36 implants 742 of varying sizes and thicknesses. With reference to figure 29, it will be appreciated that implants 742 are provided in six sizes with each implant size being provided in three different thicknesses. Accordingly, a method for selecting an implant for a particular joint will typically include the step of determining the appropriate implant size and the step of determining an appropriate implant thickness.

In the embodiment of figure 29, each size has been assigned an identifying character 744. In some embodiments of the present invention, identifying character 744 can be one or more arbitrarily selected numbers, letters or combinations of letters and numbers. In the embodiment of figure 29, each thickness has been assigned an identifying characteristic 748. In the exemplary embodiment of figure 29, each identifying characteristic 748 comprises a color. In other words, each implant having a first thickness will be red in color in this exemplary embodiment. Implants having a second thickness and a third thickness will be yellow and blue respectively, in this exemplary embodiment.

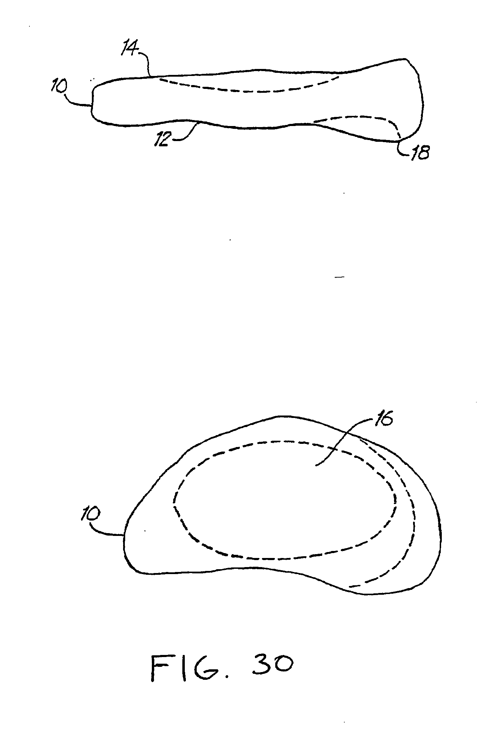

Figure 30 shows a top and side perspective of a preferred preformed knee implant (10) prepared using an *ex vivo* mold according to the present invention. The implant provides a first major surface (12) adapted to be positioned upon the tibial surface, and a generally planar second major surface (14) adapted to be positioned against the femoral condyle. In a typical embodiment, the second major surface, in turn, is preferably provided with a femoral glide path (16) to facilitate its performance *in situ*, in the form of a generally central (e.g., oval) depression about 0.5 mm, or more preferably about 1 mm to about 5mm deep at its lowest point (2 mm as shown) and about 20 mm, and more preferably about 30 mm to about 50 mm in length by 10 mm to 30 mm in width (40 mm by 20 mm as shown). Those skilled in the art, given the present description, will readily determine the actual dimensions for optimal use, in both absolute and relative terms, depending on such factors as the actual joint size and desired results (e.g., angular correction). As shown, the implant is also provided with a tibial projection (18), adapted to catch the posterior portion of the tibial plateau by extending over the rim of the tibial plateau distally. The body of the implant can have dimensions on the order of between about 35 mm, and preferably about 40 mm to about 60 mm in the anterior-posterior dimension, between about 20 mm, and preferably 30 mm to about 35 mm, or even about 40 mm in the medial-lateral dimension, and a maximum thickness (at the posterior lip of between about 8 mm, more preferably about 10 mm, and about 20 mm, or about 2 mm to about 4 mm (e.g., 3 mm) greater than the thickness of the implant at the center. As a result, it can be seen that fixation is accomplished by effectively capping the tibial plateau with one or more projections extending distally over the rim of the plateau.

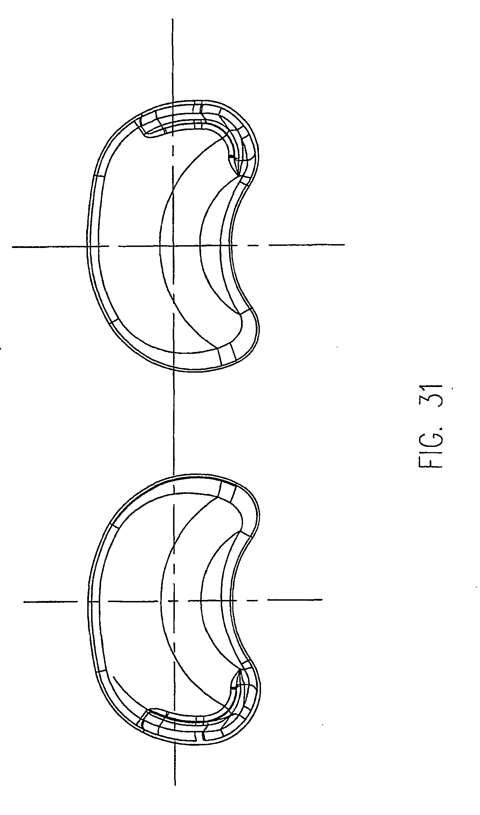

Figure 31 shows side-by-side top plan views and of corresponding implants for the left and right knees. The preformed knee implants of figure 31 include a first major surface adapted to be positioned upon the tibial surface, and a generally planar second major surface adapted to be positioned against the femoral condyle. The second major surface is shown having a femoral glide path surface to facilitate its performance *in situ,* adapted to form a generally central depression having the dimensions described herein. The glide path is fully formed in situ, by a suitable combination of both shaping and repositioning of the femoral condyle in the manner described herein.

An implant of the type shown provides various benefits, including the correction of varus deformities, based in significant part upon the presence and configuration of the posterior mesial lip, and the cutout (kidney bean shaped) for the intercondylar eminence. The tibial projection is adapted to catch the posterior portion of the tibial plateau. The implant itself has dimensions as provided herein, and can be provided using one of a collection of molds of multiple sizes and/or styles in accordance with the various parameters of the present invention. A kit can be provided containing implants of various sizes, e.g., varying by 1 mm increments in thickness and providing a range of anterior to posterior dimensions. Such implants can also be provided having bottoms of various shapes, e.g., either a flat or curved bottom, and for either the left or right knee.

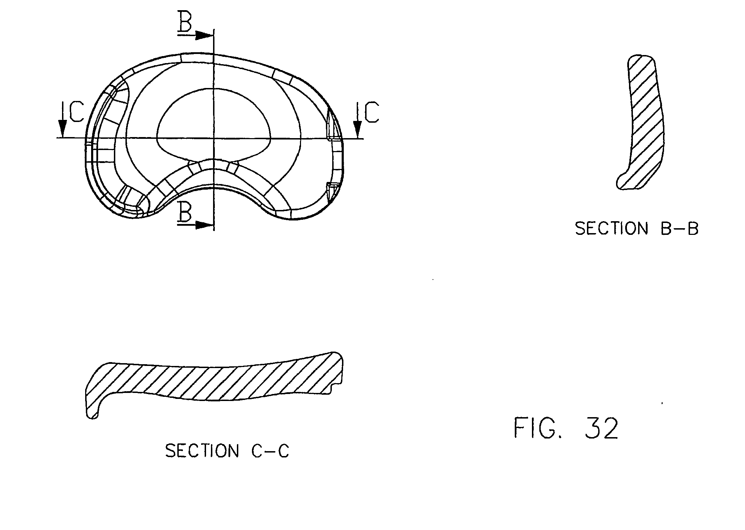

A further preferred embodiment is shown with respect to Figure 32, in which the posterior lip is shown as proceeding in a mesial direction so as to occupy the posterior cruciate ligament sulcus when positioned *in vivo.* Figure 32 includes a top view, a sectional view taken section line B-B and a section view taken along section line C-C. Together with the mesial lip, the top of the implant provides a desired glide path, while the bottom of the implant is provided with a convex bottom configuration in order to better conform to the cavity of an arthritic posterior tibial plateau.

In the embodiment shown, the mesial rim is raised approximately 2mm, reaching highest point as it approaches the intercondylar eminence, in order to add to stability and maintain the overall thickness of the implant. In addition to contributing to the desired glide path, the anterior portion of the implant is also provided with additional bulk providing it with a slightly wedge-shaped anterior region sloping from the base of the posterior lip to the anterior edge, in order to improve its posterior directional stability.

In an advantageous embodiment, a slight amount of material is removed from the anterior mesial portion to form a cavity that is dimensioned so as to reduce the likelihood that the implant will impinge on the fermoral condyle and perhaps even the medial border of the patella. In the posterior region, the lip is lengthened a bit, so that with a 54 mm implant (measured as the longest dimension from the most anterior point to the upper inner radius or the posterior lip), the lip is shown as being on the order of 6 mm in height. Commensurate with the lengthening of the posterior lip, additional bulk is removed from the top, permitting the glide path to remain open in a posterior direction. This configuration allows for more complete flexion, lessening the extent to which the implant might impinge on the cartilage of the posterior medial femoral condyle, together with improved retention within the joint.

In use, the tibial plateau is congruent to the bottom of the implant. The tibial plateau is itself prepared to provide good fit on the tibial side of the implant, while the femoral surface of the implant can be smoothed and opened up so as to be amenable with most any femoral geometry.

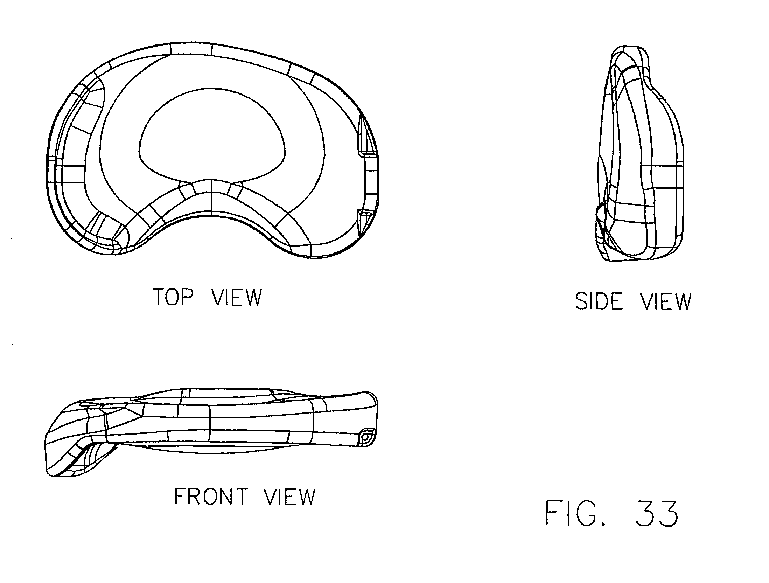

Figure 33 includes three additional plan views the implant shown in the previous figure. Figure 33 includes a top plan view that is similar to the top view of the previous figure. Figure 33 also includes a side view showing the posterior lip of the implant, and a front view.

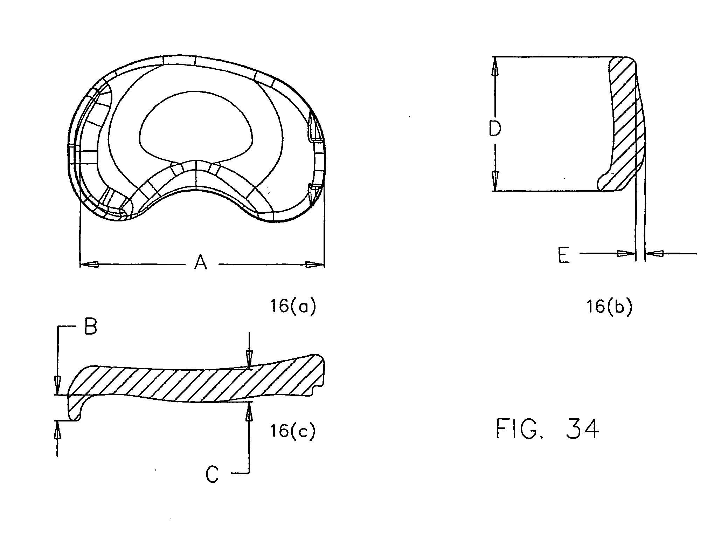

Figure 34 shows various views of an implant in accordance with an exemplary embodiment of the present invention. In figure 34, a distance A is shown extending from the most anterior point of the implant to the upper inner radius of the posterior lip of the implant. A distance B is also illustrated in figure 34. Distance B can be described as the height of the posterior lip. Distances C, D, and E are also illustrated in figure 34.

The dimensions of the implant can be scaled to fit a particular size of patient. In one exemplary embodiment of the present invention, distance A is about 54.0 mm, distance B is about 5.6 mm, distance C is about 7.0 mm, distance D is about 29.2 mm, and distance E is about 2.1 mm.

In some advantageous embodiments of the present invention, distance A is, for example, between about 30 mm and about 60 mm.

In some advantageous embodiments of the present invention, distance B is, for example, between about 1 mm and about 10 mm.

In some advantageous embodiments of the present invention, distance C is, for example, between about 1 mm and about 10 mm.

In some advantageous embodiments of the present invention, distance D is, for example, between about 10 mm and about 40 mm.

In some advantageous embodiments of the present invention, distance E is, for example, between about 0.2 mm and about 4 mm.

Implants such as those described above are preferably used in a method that includes first determining the proper implant thickness needed to match physiological valgus. The surgeon prepares the site arthroscopically, removing excess cartilage and removing the medial meniscus to the medial ring, using a portal of about 1 cm in order to provide suitable arthroscopic access while maintaining the presence of fluid in the joint. The implant can be initially molded ex vivo, using a mold selected from those available and including one or more embedded or attached fixation portions (e.g., anterior sutures or tabs), at which time it is inserted into the knee. The surgeon will then typically feel the implant once in position, to confirm that the implant is properly seated, and will extend the knee to provide varus stress on the lower leg, obtaining congruency as the implant continues to cure by finally molding both surfaces of the implant (to both the tibial surface and condyle, respectively).

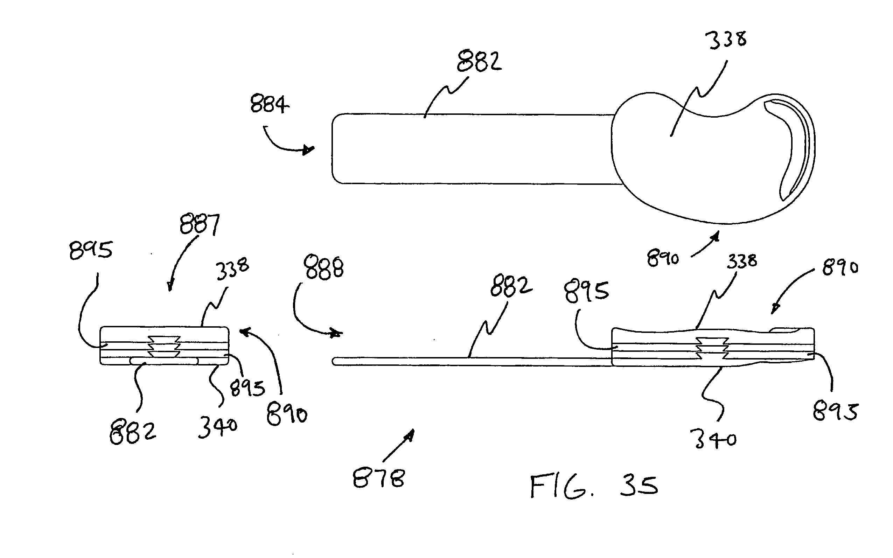

Figure 35 includes a number of views showing an implant template 878 in accordance with the present invention. More particularly, figure 35 includes a top view 884, a side view 888, and an end view 887. With reference to figure 35, it will be appreciated the implant template 878 comprises an implant-like portion 890 and a handle 882. Implant-like portion 890 comprises a first member 338 adapted to be positioned upon the tibial plateau of a tibia, and a second member 340 adapted to be positioned against the medial condyle of a femur. As shown in figure 35, implant-like portion 890 also comprises a plurality of shims 895.

With reference to figure 35, it will be appreciated that a thickness of implant-like portion 890 of implant template 878 can be varied by changing the quantity and/or thickness of the shims 895 disposed between first member 338 and second member 340. In some useful embodiments of the present invention, shims 895 can be slid between first member 338 and second member 340 from the handle end of implant template 878. In some particularly useful embodiments of the present invention, shims 895 can be slid between first member 338 and second member 340 from the handle end of implant template 878 while implant-like portion 890 is disposed between a femur and a tibia.

Numerous characteristics and advantages of the invention covered by this document have been set forth in the foregoing description. It will be understood, however, that this disclosure is, in many respects, only illustrative. Changes can be made in details, particularly in matters of shape, size and ordering of steps without exceeding the scope of the invention. The invention's scope is, of course, defined by the appended claims.

## Claims

1. An interpositional implant system for the creation or modification of an orthopedic joint within a mammalian body, the system (154) comprising one or more apparatuses for each of the steps for: a) preparing the joint to receive the interpositional implant (156, 158, 160, 254), wherein the apparatus includes a smoothing device for preparing one or more surfaces within a joint site; b) determining an appropriate implant size for a particular joint (162), wherein the apparatus includes a caliper adapted for measuring one or more joint dimensions; c) determining an appropriate implant thickness (170, 172), wherein the apparatus for determining an appropriate implant thickness includes a plurality of trial implants of one or more varying dimensions and/or configurations; and d) inserting the implant into the joint (164), wherein the apparatus includes a plurality of opposing jaws.

2. A system according to claim 1, further comprising one or more apparatuses for securing the implant within the joint to a desired extent using a plurality of tethers.

3. A system according to claims 1 or 2, wherein the smoothing device for preparing one or more surfaces within an articulating joint site, comprises a substantially flat, straight or curved, blade having a proximal portion adapted to be hand held and/or attached to a powered surgical instrument, and a distal portion having at least one major surface provided with a texture adapted to smooth cartilage within the joint site.

4. A system according to claim 3 wherein the blade is fabricated from surgical stainless steel, and a distal portion of the blade is textured by providing either a plurality of closely spaced holes extending through the width of the blade or a plurality of pegs or ridges positioned upon the blade.

5. A system according to any one of the preceding claims, wherein the caliper comprises a device adapted for use in the knee in order to determine a dimension between the anterior and posterior edges of the tibial surface, while also providing a suitable depth measurement of the tibial depression at a point approximately midway between the raised anterior and posterior edges of the tibial plateau.

6. A system according to claim 5 wherein the caliper is adapted for measuring one or more dimensions associated with the knee, including to measure one or more dimensions selected from the group consisting of an anterior-posterior dimension, a medial-lateral dimension, and a height/depth dimension.

7. A system according to any one of the preceding claims wherein the the plurality of trial implants comprises a plurality of knee implants of varying thickness to account for the ligament laxity in a particular knee joint and incorporate a design feature selected from the group consisting of number coded, bar coded, shape coded, tactile coded and/or visually coded.

8. A system according to any one of the preceding claims, wherein the plurality of opposing jaws for inserting the implant further comprises a handle and a locking mechanism adapted to secure the jaws in position upon an implant.

9. A system according to any one of the preceding claims, further comprising one or more ancillary components adapted to secure an implant in the body.

10. A system according to claim 2 wherein:
a. the smoothing device for preparing one or more surfaces within an articulating joint site, the device comprises a substantially flat, straight or curved, blade having a proximal portion adapted to be hand held and/or attached to a powered surgical instrument, and a distal portion having at least one major surface provided with a texture adapted to smooth cartilage within the joint site,
b. the caliper is adapted for use in the knee in order to determine a dimension between the anterior and posterior edges of the tibial surface, while also providing a suitable depth measurement of the tibial depression at a point approximately midway between the raised anterior and posterior edges of the tibial plateau,
c. the apparatus for determining joint thickness comprises a plurality of trial implants of one or more varying dimensions and/or configurations,
d. the plurality of opposing jaws, further comprises a handle and a locking mechanism adapted to secure the jaws in position upon an implant,
e. the plurality of tethers comprise one or more ancillary components integrated into, and partially extending from, the implant to provide fixation, and
f. one or more intepositional implants wherein at least one implant comprises a partially or fully preformed metallic and/or polymeric components, adapted to be inserted and positioned at a joint site to provide an implant having at least one major surface in apposition to supporting bone, and at least a second major surface in apposition to opposing bone.

11. A system according to claim 3, wherein the blade is adapted for use with one or more surfaces of the bones in the knee joint and the device is adapted for use in smoothing the condylar surface.

12. A system according to claim 3, wherein the blade is fabricated from surgical stainless steel, a distal portion of the blade is textured by providing either a plurality of closely spaced holes extending through the width of the blade or a plurality of pegs or ridges positioned upon the blade, the device is adapted for use in a reciprocating saw instrument, and fabricated to retain a predetermined curved shape, the device has an overall length of between about 100 mm and 150 mm, with a substantially distal portion having a length of between about 20 mm and about 40 mm, the blade width is between about 5 mm and about 10 mm, and has a thickness of between about 0.3 mm and about 5 mm, and the proximal portion of the device is provided in the form of generally circular shaft, adapted to be fixably and releasably positioned within a powered surgical instrument.

13. A system according to claim 12, wherein the powered surgical instrument is adapted to operate the blade at an excursion distance of between about 0.5 mm and about 10 mm.

14. The system of Claims 1 or 2, wherein the caliper is adapted for measuring one or more dimensions associated with the knee.

15. The system according to claim 14, wherein the caliper is adapted to measure one or more dimensions selected from the group consisting of an anterior-posterior dimension, a medial-lateral dimension, and a height/depth dimension and the device is adapted for use in the knee and can be used to determine a dimension between the anterior and posterior edges of the tibial surface, while also providing a suitable depth measurement of the tibial depression at a point approximately midway between the raised anterior and posterior edges of the tibial plateau.

16. The system according to claim 14, wherein the caliper comprises a handle assembly and a gauge portion adapted to engage the posterior edge of the tibial plateau and without interference from the femoral condyle, further comprising a slide having a raised contact end portion which translates back and forth relative to a rule that can be positioned against the anterior portion of the tibia, further comprising a probe positioned along the length of the rule, and optionally movable laterally thereto, in order to measure the depth of any indentation, or bowl shape that the tibial surface may have, wherein the probe is mounted on a slide, moveable longitudinally with the axis of the rule, to permit it to be adjusted to make depth measurements in various locations, and the anterior-posterior dimension of the tibial surface can be read from the rule as the distance between the point contacting the posterior tibial surface edge and a point contacting the anterior edge.

17. The system according to claim 14 comprising a caliper adapted for measuring one or more dimensions associated with the knee, including to measure one or more dimensions selected from the group consisting of an anterior-posterior dimension, a medial-lateral dimension, and a height/depth dimension.

18. The system according to claims 1 or 2, wherein the plurality of opposing jaws is adapted to firmly hold an interpositional knee implant, and further comprises a handle and a locking mechanism adapted to secure the jaws in position upon an implant, the first and second jaws are pivotally coupled to the handle, and further comprising a portion adapted to bias the handle in an open position.

19. The system according to claims 1 or 2, wherein the plurality of opposing jaws is adapted to hold an anterior portion of an implant while a posterior portion of the implant is inserted between a medial condyle of a femur and tibial plateau of a tibia.

20. The system of claim 2, which includes a plurality of tethers having one or more ancillary components adapted to secure an implant.

21. The system according to claim 20, wherein at least one ancillary component is integrated into, and partially extending from, the implant to provide anterior fixation.

22. A system according to claim 21, wherein the ancillary component comprises one or more protrusions adapted to be attached to either soft tissue and/or bone at the joint site to improve fixation, and the protrusions are selected from the group consisting of sutures and/or fibrous biomaterials integrally formed with the preformed component itself, and one or more separate components for securing the implant to the joint site, selected from the group consisting of adhesives, sutures, pins, staples, screws, and combinations thereof.

23. A system according to claims 1 or 2, further comprising one or more intepositional implants.

24. A system according to claim 23, wherein at least one implant comprises a partially or fully preformed metallic and/or polymeric components, adapted to be inserted and positioned at a joint site to provide an implant having at least one major surface in apposition to supporting bone, and at least a second major surface in apposition to opposing bone, the implant comprises a knee implant, the implant provides a femoral glide path and convexity of the tibial surface of the implant, together with a posterior mesial lip, the polymeric components are provided in the form of a single preformed component comprising a biomaterial partially or completely cured in an *ex vivo* mold, the implant comprises tibial projection(s) adapted to catch the posterior portion of the tibial plateau by extending over the rim of the tibial plateau distally, and the preformed component has dimensions on the order of between about 30 to about 60 mm in the anterior-posterior dimension, between about 20 mm to about 40 mm in the medial-lateral dimension, and a maximum thickness, at the posterior lip, of between about 8 mm and about 20 mm, or about 3mm to about 10 mm greater than the thickness of the implant at the center.

25. A system according to claim 24, wherein the implant further comprises at least one ancillary component integrated into, and partially extending from, the implant to provide anterior fixation.

## Patentansprüche

1. Zwischenpositionierungs-Implantierungssystem zur Schaffung und Änderung eines orthopädischen Gelenks bei einem Säugetierkörper,
**dadurch gekennzeichnet,**
**dass** dieses System (154) eine oder mehrere Vorrichtungen für jeden der folgenden Schritte umfasst
a) das Gelenk wird derart präpariert, dass es das Zwischenpositionierungs-Implantationssystem (156, 158, 160, 254) aufnimmt, wobei die Vorrichtung eine Glättungsvorrichtung zum Präparieren von einer oder mehreren Oberflächen innerhalb einer Gelenkstelle aufweist,
b) eine geeignete Implantationsgröße für eine besondere Verbindung (162) wird festgelegt, wobei die Vorrichtung einen Taster aufweist, der für die Messung von einer oder mehreren Verbindungsdimensionen ausgebildet ist,
c) eine geeignete Implantatdicke (170, 172) wird festgelegt, wobei die Vorrichtung zur Festlegung einer geeigneten Implantatdicke eine Vielzahl von Versuchsimplantaten mit einer oder mehreren verschiedenen Dimensionen und/oder Konfigurationen aufweist, und
d) das Implantat wird in das Gelenk (164) eingefügt, wobei die Vorrichtung eine Vielzahl von gegenüberliegenden Klauen aufweist.

2. System nach Anspruch 1,
**dadurch gekennzeichnet,**
**dass** es ein oder mehrere Vorrichtungen zur Befestigung des Implantats im Gelenk im gewünschten Ausmaß umfasst, wobei eine Vielzahl von Schnüren verwendet wird.

3. System nach Anspruch 1 oder 2,
**dadurch gekennzeichnet,**
**dass** die Glättungsvorrichtung zum Präparieren einer oder mehrerer Oberflächen einer gebogenen Gelenkstelle ein im Wesentlichen flaches, gerades oder gebogenes Blatt umfasst, das einen nahen Teil, der für die Handhaltung ausgebildet und/oder an einem stromgespeisten, chirurgischen Gerät angeschlossen ist, und einen fernen Teil aufweist, der mindestens eine Hauptfläche umfasst, die mit einer Struktur derartiger Ausbildung versehen ist, dass der Knorpel in der Gelenkstelle geglättet wird.

4. System nach Anspruch 3,
**dadurch gekennzeichnet,**
**dass** das Blatt aus medizinischem, rostfreien Stahl hergestellt ist und dass ein ferner Teil des Blatts dadurch strukturiert ist, dass entweder eine Vielzahl von geschlossenen, über die Breite des Blatts verteilten Löchern oder eine Vielzahl von auf dem Blatt angeordneten Stiften oder Rippen vorgesehen ist.

5. System nach einem der vorhergehenden Ansprüche,
**dadurch gekennzeichnet,**
**dass** der Taster eine Vorrichtung aufweist, die für die Verwendung im Knie ausgebildet ist, um eine Dimension zwischen der Vorderkante und der Hinterkante der Schienbeinfläche festzustellen, während auch eine geeignete Messung der Tiefe der Schienbeinschwächung an einer Stelle vorgesehen wird, die etwa in der Mitte zwischen der sich aus der Schienbeinfläche erhebenden Vorderkante und Hinterkante liegt.

6. System nach Anspruch 5,
**dadurch gekennzeichnet,**
**dass** der Taster derart ausgebildet ist, dass eine oder mehrere dem Knie zugeordnete Dimensionen gemessen werden können, wobei die Messung einer oder mehrerer Dimensionen eingeschlossen ist, die aus der Gruppe ausgewählt ist bzw. sind, die eine Vorderkannten-Hinterkanten-Dimension, eine Mitte-Seiten-Dimension und eine Höhe-Tiefen-Dimension umfasst.

7. System nach einem der vorhergehenden Ansprüche,
**dadurch gekennzeichnet,**
**dass** die Vielzahl der Schienbeinimplantate eine Vielzahl von Knieimplantaten unterschiedlicher Dicke aufweist, um die Bindungslockerheit in einem besonderen Kniegelenk zu berücksichtigen und um ein Designmerkmal einzuschließen, das aus der Gruppe ausgewählt ist, die aus einer Nummemkodierung; einer Strichkodierung, einer Formkodierung, einer Tastkodierung und/oder einer optischen Kodierung besteht.

8. System nach einem der vorhergehenden Ansprüche,
**dadurch gekennzeichnet,**
**dass** die Vielzahl von gegenüberliegenden Klauen zur Einfügung des Implantats ferner einen Handgriff und eine Verschlussvorrichtung umfasst, die derart ausgebildet ist, dass sie die Klauen an Ort und Stelle auf einem Implantat befestigt.

9. System nach einem der vorhergehenden Ansprüche,
**dadurch gekennzeichnet,**
**dass** es ferner ein oder mehrere Hilfskomponenten aufweist, die derart ausgebildet sind, dass sie ein Implantat am Körper befestigen.

10. System nach Anspruch 2,
**dadurch gekennzeichnet, dass**
a) die Glättungsvorrichtung zum Präparieren einer oder mehrerer Oberflächen einer gebogenen Gelenkstelle ein im Wesentlichen flaches, gerades oder gebogenes Blatt umfasst, das einen nahen Teil, der für die Handhaltung ausgebildet und/oder an einem stromgespeisten, chirurgischen Gerät angeschlossen ist, und einen fernen Teil aufweist, der mindestens eine Hauptfläche umfasst, die mit einer Struktur derartiger Ausbildung versehen ist, dass der Knorpel in der Gelenkstelle geglättet wird,
b) der Taster eine Vorrichtung aufweist, die für die Verwendung im Knie ausgebildet ist, um eine Dimension zwischen der Vorderkante und der Hinterkante der Schienbeinfläche festzustellen, während auch eine geeignete Messung der Tiefe der Schienbeinschwächung an einer Stelle vorgesehen wird, die etwa in der Mitte zwischen der sich aus der Schienbeinfläche erhebenden Vorderkante und Hinterkante liegt,
c) die Vorrichtung zur Feststellung der Gelenkdicke eine Vielzahl von Versuchsimplantaten mit einer oder mehreren veränderlichen Dimensionen und/oder Konfigurationen aufweist,
d) die Vielzahl von gegenüberliegenden Klauen ferner einen Handgriff und eine Verschlussvorrichtung umfasst, die derart ausgebildet ist, dass sie die Klauen an Ort und Stelle auf einem Implantat befestigt,
e) die Vielzahl von Schnüren ein oder mehrere Hilfskomponenten aufweist, die im Implantat integriert sind und teilweise vom Implantat ausgehen, um eine Befestigung vorzusehen, und
f) ein oder mehrere Zwischenpositionierungs-Implantate, von denen mindestens ein Implantat eine teilweise oder ganz ausgebildete Metall- und/oder Polymerkomponente aufweist, derart ausgebildet sind, dass sie an einer Gelenkstelle eingefügt und positioniert werden können, um ein Implantat vorzusehen, das mindestens eine Hauptfläche in Apposition zum stützenden Knochen und mindestens eine zweite Hauptfläche in Apposition zum entgegengesetzten Knochen aufweist.

11. System nach Anspruch 3,
**dadurch gekennzeichnet,**
**dass** das Blatt für die Verwendung mit einem oder mehreren Oberflächen der Knochen im Kniegelenk und die Glättungsvorrichtung für die Verwendung zum Glätten der Condylarfläche ausgebildet ist.

12. System nach Anspruch 3,
**dadurch gekennzeichnet,**
**dass** das Blatt aus medizinischem, rostfreien Stahl hergestellt ist, dass ein ferner Teil des Blatts dadurch strukturiert ist, dass entweder eine Vielzahl von geschlossenen, über die Breite des Blatts verteilten Löchern oder eine Vielzahl von auf dem Blatt angeordnete Stiften oder Rippen vorgesehen sind, dass die Glättungsvorrichtung für die Verwendung in einer hin- und hergehenden Säge ausgebildet und derart hergestellt ist, dass eine vorbestimmte, gebogene Form gewonnen wird, dass die Glättungsvorrichtung eine Gesamtlänge von etwa 100-150mm mit einem im Wesentlichen fernen Teil mit einer Länge von etwa 20-40mm aufweist, wobei die Blattbreite etwa 5-10mm und die Blattdicke etwa 0,3-5mm beträgt, und dass der nahe Teil der Glättungsvorrichtung die Form einer generell runden Welle aufweist, die derart ausgebildet ist, dass sie fest oder entfernbar in einem stromgespeisten, medizinischem Instrument angeordnet ist.

13. System nach Anspruch 12,
**dadurch gekennzeichnet,**
**dass** das stromgespeiste, medizinische Instrument derart ausgebildet ist, dass es das Blatt bei einem Ausschlagsabstand von etwa 0,5-10mm betreibt.

14. System nach Anspruch 1 oder 2,
**dadurch gekennzeichnet,**
**dass** der Taster für die Messung einer oder mehrerer dem Knie zugeordneter Dimensionen ausgebildet ist.

15. System nach Anspruch 14,
**dadurch gekennzeichnet,**
**dass** der Taster für die Messung einer oder mehrerer Dimensionen ausgebildet ist, die aus der Gruppe ausgewählt ist bzw. sind, die eine Vorderkanten-Hinterkanten-Dimension, eine Mitte-Seiten-Dimension und eine Höhen-Tiefen-Dimension umfasst, und dass der Taster für die Verwendung im Knie vorgesehen ist und dazu verwendet werden kann, eine Dimension zwischen der Vorderkante und der Hinterkante der Schienbeinfläche festzustellen, während auch eine geeignete Messung der Tiefe der Schienbeinschwächung an einer Stelle vorgesehen ist, die etwa in der Mitte zwischen der sich aus der Schienbeinfläche erhebenden Vorderkante und Hinterkante liegt.

16. System nach Anspruch 14,
**dadurch gekennzeichnet,**
**dass** der Taster eine Handgriffanordnung und einen Kalibrierungsteil umfasst, der derart ausgebildet ist, dass er die Hinterkante der Schienbeinebene erfasst und der ohne Störung durch das Condyl des Oberschenkelknochens arbeitet, dass der Taster ferner ein Gleitstück mit einem vorspringenden Kontaktendteil aufweist, das in Bezug auf ein Lineal vor- und zurückschiebbar ist, das gegen den Vorderteil des Schienbeinknochens positionierbar ist, und dass der Taster ferner einen Sensor aufweist, der längs des Lineals positioniert und wahlfrei zu diesem seitlich bewegbar ist, um die Tiefe eines Kennzeichens oder einer Schüsselform, die die Schienbeinknochenoberfläche aufweisen kann, zu messen, wobei die Sonde auf einem Gleitstück befestigt ist, das längs der Achse des Lineals bewegbar ist, damit das Gleitstück für die Tiefenmessungen an verschiedenen Stellen eingestellt werden kann und damit die Vorderkanten-Hinterkanten-Dimension der Schienbeinknochenoberfläche vom Lineal als Abstand zwischen dem die Hinterkante der Schienbeinknochenoberfläche kontaktierenden Punkt und einem die Vorderkante kontaktierenden Punkt abgelesen werden kann.

17. System nach Anspruch 14,
**dadurch gekennzeichnet,**
**dass** es einen Taster aufweist, der für die Messung einer oder mehrerer dem Knie zugeordneter Messungen ausgebildet ist, wobei die Messung einer oder mehrerer Dimensionen eingeschlossen ist, die aus der Gruppe ausgewählt sind, die aus einer Vorderkanten-Hinterkanten-Dimension, einer Mitten-Seiten-Dimension und einer Hühen-Tiefen-Dimension besteht.

18. System nach Anspruch 1 oder 2,
**dadurch gekennzeichnet,**
**dass** die Vielzahl der gegenüberliegenden Klauen für das Festhalten eines Zwischenpositionierungs-Knieimplantats ausgebildet ist, dass das System ferner einen Handgriff und eine Verschlussvorrichtung aufweist, die für die Befestigung der Klauen in der Position auf einem Implantat ausgebildet ist, wobei die erste Klaue und die zweite Klaue mit dem Handgriff drehbar verbunden sind, und dass das System einen Teil aufweist, der für das Halten des Handgriffs in einer offenen Position ausgebildet ist.

19. System nach Anspruch 1 oder 2,
**dadurch gekennzeichnet,**
**dass** die Vielzahl der gegenüberliegenden Klauen für das Halten eines Vorderteils eines Implantats ausgebildet ist, während ein Hinterteil des Implantats zwischen einem medialen Condyl eines Oberschenkelknochens und einer Schienbeinebene eines Schienbeins eingefügt ist.

20. System nach Anspruch 2,
**dadurch gekennzeichnet,**
**dass** eine Vielzahl von Schnüren mit einer oder mehreren Hilfskomponenten vorgesehen ist, die für die Befestigung eines Implantats ausgebildet sind.

21. System nach Anspruch 20,
**dadurch gekennzeichnet,**
**dass** mindestens eine Hilfskomponente mit dem Implantat integriert ist und teilweise aus diesem hervorsteht, um eine vordere Befestigung vorzusehen.

22. System nach Anspruch 21,
**dadurch gekennzeichnet,**
**dass** die Hilfskomponente ein oder mehrere Vorsprünge aufweist, die für die Befestigung von entweder einem weichen Gewebe und/oder eines Knochens an der Gelenkstelle ausgebildet ist, um die Befestigung zu verbessern, wobei die Vorsprünge aus der Gruppe ausgewählt sind, die aus Nähten und/oder Biofasermaterial besteht, die mit der vorgeformten Komponente selbst integriert gebildet sind, und dass das System ein oder mehrere getrennte Komponenten zur Befestigung des Implantats an der Gelenkstelle aufweist, wobei diese Komponenten aus der Gruppe ausgewählt sind, die aus Klebstoffen, Nähten, Stiften, Klammern, Schrauben und Kombinationen daraus besteht.

23. System nach Anspruch 1 oder 2,
**dadurch gekennzeichnet,**
**dass** es ein oder mehrere Zwischenpositionierungs-Implantate umfasst.

24. System nach Anspruch 23,
**dadurch gekennzeichnet,**
**dass** mindestens ein Implantat eine teilweise oder ganz vorgeformte, metallische oder polymere Komponente aufweist, die für die Einfügung und Positionierung an einer Gelenkstelle ausgebildet ist, um ein Implantat zu schaffen, das mindestens eine Hauptfläche in Apposition zum stützenden Knochen und mindestens eine zweite Hauptfläche in Apposition zum entgegengesetzten Knochen aufweist, dass das Implantat ein Knieimplantat aufweist, dass das Implantat einen Gleitweg für den Oberschenkelknochen und eine konvexe Form der Schienbeinoberfläche des Implantats zusammen mit einer hinteren Mesiallippe aufweist, dass die polymeren Komponenten in Form einer einzelnen, vorgeformten Komponente aus einem Biomaterial vorgesehen sind, das teilweise oder ganz in einer *ex-vivo*-Gießform ausgehärtet ist, dass das Implantat einen Schienbeinvorsprung (Schienbeinvorsprünge) aufweist, der für das Aufnehmen des Hinterteils der Schienbeinebene ausgebildet ist und am fernen Ende über den Rand der Schienbeinebene hinausragt, und dass die vorgeformte Komponente Dimensionen aufweist, die in der Größenordnung von etwa 30-60mm in Bezug auf die Vorderkanten-Hinterkanten-Dimension, von etwa 20-40mm in Bezug auf die Mitten-Seiten-Dimension und eine Maximaldicke an der Hinterlippe von etwa 8-20mm oder um 3-10mm größer als die Dicke des Implantats in der Mitte liegen.

25. System nach Anspruch 24,
**dadurch gekennzeichnet,**
**dass** das Implantat ferner mindestens eine Hilfskomponente aufweist, die mit dem Implantat integriert ist und sich teilweise von diesem weg erstreckt, um die Vorderbefestigung zu verbessern.

## Revendications

1. Système d'implant d'interposition destiné à la création ou à la modification d'une articulation orthopédique d'un corps de mammifère, le système (154) comprenant un ou plusieurs appareils destinés à chacune des étapes consistant à : a) préparer l'articulation à recevoir l'implant d'interposition (156, 158, 160, 254), l'appareil comprenant un dispositif de lissage destiné à préparer une ou plusieurs surfaces situées à l'intérieur du site de l'articulation ; b) déterminer une dimension d'implant appropriée à une articulation particulière (162), l'appareil comprenant un pied à coulisse apte à mesurer une ou plusieurs dimensions de l'articulation ; c) déterminer une épaisseur d'implant appropriée (170, 172), l'appareil destiné à déterminer une épaisseur d'implant appropriée comprenant une pluralité d'implants d'essai dont une ou plusieurs dimensions et/ou configurations varient ; et d) insérer l'implant dans l'articulation (164), l'appareil comprenant une pluralité de mâchoires se faisant face.

2. Système selon la revendication 1 comprenant en outre un ou plusieurs dispositifs destinés à fixer l'implant dans l'articulation dans la mesure souhaitée en utilisant une pluralité d'attaches.

3. Système selon la revendication 1 ou 2, dans lequel le dispositif de lissage destiné à la préparation d'une ou de plusieurs surfaces à l'intérieur d'un site d'articulation, comprend une lame substantiellement plate, rectiligne ou courbe comportant une partie proximale apte à être tenue à la main et/ou à être fixée à un instrument chirurgical alimenté en énergie et une partie distale ayant au moins une surface importante dotée d'une texture apte à lisser le cartilage situé dans le site de l'articulation.

4. Système selon la revendication 3, dans lequel la lame est fabriquée en acier inoxydable chirurgical, et une partie distale de la lame est texturée en ménageant soit une pluralité de trous espacés de façon serrée s'étendant sur la largeur de la lame, soit une pluralité d'aspérités ou de nervures positionnées sur la lame.

5. Système selon l'une quelconque des revendications qui précèdent, dans lequel le pied à coulisse comprend un dispositif apte à être utilisé dans le genou afin de déterminer une distance entre les bords antérieur et postérieur de la surface du tibia tout en procurant également une mesure de profondeur adéquate du creux du tibia en un point situé approximativement à mi-chemin entre les bords antérieur et postérieur relevés du plateau tibial.

6. Système selon la revendication 5, dans lequel le pied à coulisse est apte à mesurer une ou plusieurs dimensions en rapport avec le genou, comprenant la mesure d'une ou de plusieurs dimensions choisies dans le groupe consistant en une dimension antérieure-postérieure, une dimension médiane-latérale, et une dimension hauteur-profondeur.

7. Système selon l'une quelconque des revendications qui précèdent, dans lequel la pluralité d'implants d'essai comprend une pluralité d'implants destinés au genou, d'épaisseurs variées, pour tenir compte du relâchement du ligament dans une articulation du genou spécifique et elle incorpore une caractéristique de conception choisie dans le groupe constitué des codes numériques, des codes barres, des codes de forme, des codes tactiles et/ou des codes visuels.

8. Système selon l'une quelconque des revendications qui précèdent, dans lequel la pluralité de mâchoires opposées destinées à l'insertion de l'implant comprend en outre une poignée et un mécanisme de verrouillage apte à fixer les mâchoires en place sur un implant.

9. Système selon l'une quelconque des revendications qui précèdent, comprenant en outre un ou plusieurs composants subordonnés aptes à fixer un implant dans le corps.

10. Système selon la revendication 2, dans lequel :
a. le dispositif de lissage est destiné à préparer une ou plusieurs surfaces à l'intérieur du site de l'articulation, ce dispositif de lissage comprenant une lame substantiellement plate, rectiligne ou courbe, comportant une partie proximale apte à être tenue à la main et/ou fixée à un instrument chirurgical alimenté en énergie, et une partie distale ayant au moins une surface importante dotée d'une texture apte à lisser le cartilage situé dans le site de l'articulation ;
b. le pied à coulisse est apte à être utilisé dans le genou afin de déterminer une distance entre les bords antérieur et postérieur de la surface du tibia, tout en procurant également une mesure adéquate de la profondeur du creux du tibia en un point situé approximativement à mi-chemin entre les bords antérieur et postérieur relevés du plateau tibial ;
c. l'appareil destiné à déterminer l'épaisseur de l'articulation comprend une pluralité d'implants d'essai d'une ou de plusieurs dimensions et/ou configurations qui varient ;
d. la pluralité de mâchoires qui se font face comprend en outre une poignée et un mécanisme de verrouillage apte à fixer les mâchoires en place sur un implant ;
e. la pluralité d'attaches comprend un ou plusieurs composants subordonnés intégrés dans et s'étendant partiellement à partir de l'implant pour en assurer la fixation ; et
f. un ou plusieurs implants d'interposition dans lesquels au moins un implant comprend un composant en métal ou en polymère partiellement ou complètement préformé, apte à être inséré et positionné sur un site d'articulation pour procurer un implant ayant au moins une surface importante appliquée sur l'os de support et au moins une deuxième surface importante appliquée sur l'os opposé.

11. Système selon la revendication 3, dans lequel la lame est apte à être utilisée avec une ou plusieurs surfaces des os de l'articulation du genou et le dispositif est apte à être utilisé pour lisser la surface condylaire.

12. Système selon la revendication 3, dans lequel la lame est fabriquée en acier inoxydable chirurgical, une partie distale de la lame est texturée en ménageant soit une pluralité de trous espacés de façon serrée s'étendant sur la largeur de la lame, ou une pluralité d'aspérités ou de rides positionnées sur la lame, le dispositif est apte à être utilisé dans un instrument du type scie à va-et-vient et il est fabriqué de manière à maintenir une forme courbe prédéterminée, le dispositif à une longueur hors-tout comprise entre environ 100 mm et environ 150 mm, avec une partie substantiellement distale ayant une longueur comprise entre environ 20 mm et environ 40 mm, la largeur de la lame est comprise entre environ 5 mm et environ 10 mm, et elle a une épaisseur comprise entre environ 0,3 mm et environ 5 mm, et la partie proximale du dispositif est ménagée sous forme d'une tige généralement circulaire, apte à être positionnée de façon fixe et détachable dans un instrument chirurgical alimenté en énergie.

13. Système selon la revendication 12, dans lequel l'instrument chirurgical alimenté en énergie est apte à faire fonctionner la lame à une distance d'excursion comprise entre environ 0,5 mm et environ 10 mm.

14. Système selon la revendication 1 ou 2, dans lequel le pied à coulisse est apte à mesurer une ou plusieurs dimensions en rapport avec le genou.

15. Système selon la revendication 14, dans lequel le pied à coulisse est apte à mesurer une ou plusieurs dimensions choisies parmi le groupe constitué d'une dimension antérieure-postérieure, d'une dimension médiane-latérale, et d'une dimension hauteur-profondeur et le dispositif est apte à être utilisé dans le genou et il peut être utilisé pour mesurer une distance entre les bords antérieur et postérieur de la surface du tibia, tout en procurant une mesure adéquate de la profondeur du creux du tibia en un point situé approximativement à mi-chemin entre les bords antérieur et postérieur relevés du plateau tibial.

16. Système selon la revendication 14, dans lequel le pied à coulisse comprend un ensemble de poignée et une partie jauge apte à se mettre en contact avec le bord postérieur du plateau tibial et sans interférence provenant du condyle fémoral, comprenant en outre un coulisseau comportant une partie d'extrémité de contact relevée qui se déplace en translation en va-et-vient par rapport à une règle qui peut être positionnée contre la partie antérieure du tibia, comprenant en outre une sonde positionnée sur la longueur de la règle et pouvant être optionnellement déplacée latéralement, afin de mesurer la profondeur de n'importe quelle indentation, ou forme de coupe, que peut comporter la surface du tibia, la sonde étant montée sur un coulisseau, apte à être déplacé longitudinalement à l'axe de la règle, pour permettre un réglage en vue d'effectuer des mesures de profondeur à divers emplacements et la dimension antérieure-postérieure de la surface du tibia peut être lue sur la règle sous forme de la distance séparant le point de contact avec le bord postérieur de la surface du tibia et un point de contact avec le bord antérieur.

17. Système selon la revendication 14 comprenant un pied à coulisse apte à mesurer une ou plusieurs dimensions en rapport avec le genou, comprenant la mesure d'une ou de plusieurs dimensions choisies dans le groupe constitué par une dimension antérieure-postérieure, par une dimension médiane-latérale et par une dimension hauteur-profondeur.

18. Système selon la revendication 1 ou 2, dans lequel la pluralité de mâchoires qui se font face est apte à tenir fermement un implant d'interposition pour le genou et elle comprend en outre une poignée et un mécanisme de verrouillage apte à fixer les mâchoires en place sur un implant, les première et deuxième mâchoires sont accouplées de façon pivotante à la poignée et elles comprennent en outre une partie apte à pousser la poignée en position d'ouverture.

19. Système selon la revendication 1 ou 2, dans lequel la pluralité de mâchoires qui se font face est apte à maintenir une partie antérieure d'un implant pendant qu'une partie postérieure de l'implant est insérée entre le condyle médian d'un fémur et le plateau tibial d'un tibia.

20. Système selon la revendication 2 comprenant une pluralité d'attaches dotées d'un ou de plusieurs composants subordonnés aptes à fixer un implant.

21. Système selon la revendication 20, dans lequel au moins un composant subordonné est intégré dans et s'étend partiellement depuis l'implant afin de procurer une fixation antérieure.

22. Système selon la revendication 21, dans lequel le composant subordonné comprend une ou plusieurs saillies aptes à être fixées à un tissu lisse et/ou à un os lisse au niveau du site de l'articulation pour améliorer la fixation et les saillies sont choisies dans le groupe constitué de sutures et/ou de biomatériaux fibreux formés d'un seul tenant avec le composant préformé lui-même, et un ou plusieurs composants séparés destinés à fixer l'implant au site d'articulation, lesquels sont choisis dans le groupe constitué d'adhésifs, de sutures d'épingles, d'agrafes, de vis et de combinaisons de ceux-ci.

23. Système selon les revendications 1 ou 2, comprenant en outre un ou plusieurs implants d'interposition.

24. Système selon la revendication 23, dans lequel au moins un implant comprend un composant en métal et/ou en polymère partiellement ou complètement préformé, apte à être inséré et positionné au niveau d'un site d'articulation pour procurer un implant ayant au moins une surface importante appliquée à l'os de support, et au moins une deuxième surface importante appliquée à l'os opposé, l'implant comprend un implant pour le genou, l'implant procure un chemin de glissement fémoral et une convexité de la surface tibiale de l'implant en même temps qu'une lèvre médiane postérieure, les composants polymères sont fournis sous la forme d'un composant préformé unique comprenant un biomatériau partiellement ou complètement durci dans un moule *ex vivo,* l'implant comprend une(des) saillie(s) tibiale(s) apte(s) à s'accrocher sur la partie postérieure du plateau tibial en s'étendant distalement au-dessus du bord du plateau tibial, et le composant préformé a des dimensions de l'ordre compris entre environ 30 mm et environ 60 mm dans la dimension antérieure-postérieure, entre environ 20 mm et environ 40 mm dans la dimension médiane-latérale et une épaisseur maximum au niveau de la lèvre postérieure comprise entre environ 8 mm et environ 20 mm, ou supérieure d'environ 3 mm à environ 10 mm à l'épaisseur de l'implant au centre.

25. Système selon la revendication 24, dans lequel l'implant comprend en outre au moins un composant subordonné intégré dans et s'étendant partiellement depuis l'implant afin de procurer une fixation antérieure.
